# EUROPEAN PATENT SPECIFICATION

(11) **EP 1 893 997 B1**
(45) Date of publication and mention of the grant of the patent: **04.08.2010**
(21) Application number: 06785512.2
(22) Date of filing: 23.06.2006
(51) Int. Cl.: G01N 33/48, G01N 33/00, G01N 1/00

(54) **QUANTITATIVE ASSAYS FOR RAS P21 IN BODY FLUIDS**
QUANTITATIVE ASSAYS FÜR RAS P21 IN KÖRPERFLÜSSIGKEITEN
ESSAIS QUANTITATIFS DE RECHERCHE DE RAS P21 DANS LES FLUIDES CORPORELS

(30) Priority: 23.06.2005 US 694082 P
(43) Date of publication of application: 05.03.2008
(73) Proprietor: Siemens Healthcare Diagnostics Inc., Tarrytown, NY 10591 (US)
(72) Inventor: CARNEY, Walter, P., North Andover, MA 01845 (US); HAMER, Peter, J., Reading, MA 01867 (US); PIERCE, Karen, Acton, MA 01720 (US); BROWN-SHIMER, Sheryl, Boston, MA 02188 (US)
(74) Representative: Maier, Daniel Oliver
(86) International application number: PCT/US2006/024647
(87) International publication number: WO 2007/002505

(56) References cited:
- EP-A- 0 206 065
- EP-A- 0 214 520
- US-A- 5 443 956
- US-A1- 2003 219 842
- RUNDLE ANDREW ET AL: "Association between the ras p21 oncoprotein in blood samples and breast cancer" CANCER LETTERS, vol. 185, no. 1, 8 November 2002 (2002-11-08), pages 71-78, XP002486942 ISSN: 0304-3835
- FURTH M E ET AL: "MONOCLONAL ANTIBODIES TO THE P21 PRODUCTS OF THE TRANSFORMING GENE OF HARVEY MURINE SARCOMA VIRUS AND OF THE CELLULAR RAS GENE FAMILY" JOURNAL OF VIROLOGY, THE AMERICAN SOCIETY FOR MICROBIOLOGY, US, vol. 43, no. 1, 1 July 1982 (1982-07-01), pages 294-304, XP008041021 ISSN: 0022-538X
- GULBIS BEATRICE ET AL: "Immunodetection of the p21-ras products in human normal and preneoplastic tissues and solid tumors: A review" HUMAN PATHOLOGY, vol. 24, no. 12, 1993, pages 1271-1285, XP002486943 ISSN: 0046-8177
- RUNDLE A. ET AL.: 'Association between the ras p21 oncoprotein in blood samples and breast cancer' CANCER LETTERS vol. 185, 2002, pages 71 - 78

## Description

### FIELD OF THE INVENTION

The present invention is in the general area of medical genetics and in the fields of oncology and immunology. More specifically, the invention is directed to the detection and quantification of total ras p21 in body fluids, particularly serial changes of total ras p21 levels in a subject's body fluids. Further, the invention is directed to detecting and quantitating total ras p21 in conjunction with one or more other proteins, such as, oncoproteins, angiogenic factors, tumor markers, inhibitors, growth factor receptors, metastasis proteins, and tumor suppressors.

### BACKGROUND

The *ras* family of oncogenes (homologous to the rat sarcoma virus) belongs to the G protein family of cellular proteins engaged in signal transduction. The *ras* genes code for proteins that are located on the inner surface of the plasma membrane and have GTPase activity. Normally, when triggered by growth factor binding to plasma membrane receptors, ras proteins bind GTP, resulting in activation of the ras signal. The *ras* gene family has three primary members (H-*ras*, K-*ras*, and N-*ras*) that are among the most commonly activated oncogenes found in human cancer. Other *ras* gene family members include the Rho/Rac, Rab, Arf and Ran subfamilies.

The three main *ras* genes (H-*ras*, K-*ras*, and N-*ras*) encode 21 kD alpha/beta sheet proteins of 189 amino acids which are found in all eukaryotic cells on the inner surface of the plasma membrane. The isoforms (denoted H-Ras, N-Ras and K-Ras) encoded by the three *ras* genes are collectively known in the art as "p21" or "ras p21," and share a high degree of homology (>90%) as well as common downstream effectors and upstream guanine-nucleotide exchange factors (GEFS). As is true for the other members of the *ras* gene family, ras p21 is an intracellular GDP/GTP binding G protein important in cell signal transduction, converting signals from receptor tyrosine kinases in the cell membrane to the nucleus to regulate normal cell proliferation and differentiation [Pruitt and Der (2002)]. Ras p21 proteins activated by substitutions at amino acid positions 12, 13, or 61 can act as oncoproteins with an increased transforming capacity. Mutated forms of ras p21 are locked in a constitutively active state by reducing GTP hydrolysis, which results in uncontrolled signals for cell growth. When either mutated or overexpressed, p21 proteins are oncogenic. Ras p21 proteins are overexpressed in 50-60% of breast cancers and mutated in approximately 30% of all cancers, notably pancreatic and colon cancer.

Mutational activation of *ras* oncogenes has been identified in a variety of cancers as well as precursor lesions. Mutationally activated *ras* genes are uncommon in stomach, esophagus, ovary, prostate and breast tumors but are reported in 20-40% of lung carcinomas, 30% of acute myelogenous leukemia, 40-50% of colorectal tumors and thyroid tumors and > 80% of pancreatic tumors. Overall, approximately 30% of all human neoplasms have been reported to contain a *ras* gene mutation. [Adjei (2001); Bos (1989).] H-*ras* mutations are common in bladder, kidney, and thyroid carcinomas; K-*ras* mutations occur in non-small-cell lung, colorectal and pancreatic carcinomas; and N-*ras* mutations are found in melanoma, hepatocellular carcinoma, and hematologic malignancies. [Adjei (2001).] Transformation of cells by oncogenic ras mutants can increase the expression of metalloproteinases, such as gelatinase and stromelysin, which can enhance tumor metastasis. In addition, the expression of vascular endothelial growth factor (VEGF) is increased in K-ras- and H-ras transformed epithelial cells through the Raf pathway.

There has been some controversy in the art about the prognostic significance of mutated versus overexpressed ras p21. For example, Watson et al. [Breast Cancer Res. Treat., 17(3): 161-169 (Jan-Feb 1991)] reported that elevated p21 levels detected by semiquantitative Western blot were an important prognostic indicator in early breast cancer patients. In 2001, however, Karelia et al. [Int. J. Biol. Markers. 16(2): 97-104 (2001)] investigated p21 ras oncoprotein expression in colorectal cancer using immunohistochemical studies, and concluded that genetic alteration of p21 ras, not ras p21 positivity, was important in determining biological aggressiveness of colorectal cancer.

Antibodies that are used frequently to detect ras in tumors are pan reactive antibodies, which bind to all three forms of ras p21 (H-, N- and K-ras), both mutated and wild-type. For example, Sun et al., [Eur. J. Cancer, 27(12): 1646-1649 (1991)] used the pan reactive Mab Ras 11 in immunohistochemical studies to examine the significance of ras p21 in colorectal adenocarcinoma, and found ras p21 positivity to correlate with proliferative activity and to have independent prognostic value.

Although quantitative assays of ras p21 in human tissues are available, earlier described assays of ras p21 in human body fluids are at best semi-quantitative. As ras is normally expressed, it would be useful to have an indicator of an activated (upregulated and/or mutationally stimulated) ras oncoprotein pathway, such as a quantitative assay of elevated or serial changes in circulating ras p21 levels, wherein elevated levels or changes in ras p21 levels could be used to detect ras-driven oncogenesis or changes in the status of a ras-driven tumor.

Carney, W.P., US Patent No. 5,443,956 (1995) describes semi-quantitative immunoassays of total ras p21 in normal human plasma but not in corresponding sera. That Carney patent also describes the use of the pan reactive Mab Ras 17 to detect total ras p21 in ELISA assays of normal human plasma. EP 0 214 520 discloses a sandwich immunoassay for ras p21 that is of quantitative nature.

By 2000, at least two investigators had reported the presence of ras protein in the sera of lung cancer patients. In 1991, Brandt-Rauf, P.W., [J. Occup. Med., 33(9): 951-955 (Sept. 1991)] detected ras in the serum of five out of 11 lung cancer patients and two out of 21 patients with non-malignant lung diseases using immunoblots, and suggested the use of ras as a marker for the early detection of respiratory tract cancer. Anderson et al. also analyzed ras levels in the plasma of human lung cancer patients using Western blots. [Anderson et al., Mutat. Res. 349: 121-126 (1996); Anderson et al., Mutat. Res 403(1-2): 229-235 (1998); Anderson et al., Int. J. Hyg. Environ. Health. 204(1): 55-60 (Oct. 2001).] In the Anderson et al. 1996 study, ras levels were increased in 45% patients vs. 6% of normal controls. In some of the Anderson et al. studies, the primary antibody Ras 10, which detects total ras protein (reactive with normal and mutant ras) was used. Ras levels were not quantified (in picograms) in either the Brandt-Rauf (1991) or the Anderson et al. studies.

Schneider et al. [Clin. Chem. Lab. Med., 38(4): 301-305 (2000)] reported that ras p21 could not be detected in lung cancer patient sera, or in sera from any of the study groups, and therefore ras could not be considered useful as a marker for the early detection of lung cancer. Schneider et al. (2000) assayed sera of 65 male lung cancer patients, 29 non-malignant lung disease, and 44 controls, using Ab-1 (pan reactive anti-ras p21 rat Mabs from clone Y13-259) which reacts with H, K and N-ras, in three different Western blot methods. However, he could not detect ras in those serum samples, although 1 ng ras protein was measurable in standards.

Rundle et al. [Cancer Lett. 185(1): 71-78 (Nov. 8, 2002)] examined ras p21 levels in plasma from breast cancer patients, using Western blots, computer aided image analysis (integrated pixel units), and a pan reactive anti-ras antibody. Rundle et al. (2002) found detectable levels of wild-type ras in 53% of the breast cancer patients; the average levels in breast cancer patients were qualitatively higher than the average wild-type ras levels found in positive control subjects (27% of benign breast disease patients and 26% of healthy controls). Perera et al. [Arch. Toxicol., 64(5): 401-406 (1990)] studied serial changes in serum levels of 9 different oncogene protein products, including ras, both prior to and throughout the course of chemotherapy. Again, the ras determinations of both the Rundle et al. (2002) and Perera et al. (1990) assays were only semi-quantitative.

It may be critical to assay for both mutated and overexpressed ras p21 even in those cancers that are not associated with mutated ras, as overexpressed ras may also be indicative of an activated ras pathway. For example, although ras is overexpressed in 50-60% and mutated in less than 5% of breast cancers, there is considerable experimental evidence that mutationally activated ras can promote breast cancer growth and development, possibly due to activation of HER-2/neu or other oncogenes. [Eckert et al., Cancer Research, 64: 4585 - 4592 (2004).]

The inventors have discovered a quantitative immunoassay that can be used to measure serial changes in total ras p21 levels in body fluids, which changes are useful as an indicator of an activated (upregulated and/or mutationally stimulated) ras pathway. The novel immunoassay comprises the use of the pan reactive monoclonal antibodies (Mabs) Ras 17 and Ras 10 or Ras 10.2, each of which bind total ras p21 protein (both normal and mutation-activated ras p21). The Mabs Ras 17 and Ras 10 have been described previously [Carney, W.P., US Patent Nos. 5,262,523, 5,443,956, 5,081,230 and 6,200,764]; however, the use of the combination of Mabs Ras 17 and Ras 10 in an immunoassay has not been previously described.

The present invention discloses quantitative assays to detect changes in total ras p21 levels in body fluid samples to detect activated ras pathways, whether upregulated or mutationally stimulated. Such assays alone or in combination with levels of cancer markers upstream of ras (such as TIMP-1), or with levels of other proteins, may be used to select patient therapies targeted to the ras pathway. Because of the importance of ras in tumorigenesis, several approaches have been taken for developing ras-targeted anticancer therapies, such as inhibition of ras protein expression (e.g., antisense oligonucleotides), prevention of membrane localization essential for ras activation (e.g. using Ras farnesylation inhibitors) [Kohl et al. (1995); Lerner et al. (1995)], or inhibition of downstream effectors of ras (e.g., Raf serine/threonine kinases) thereby preventing the constitutive activation of the MAPK signal cascade. [Pruitt and Der (2002).] One promising candidate for a ras pathway-targeted therapy is the bis-aryl urea sorafenib (BAY-43-9006) [Wilhelm et al., Cancer Research, 64: 7099-7109 (Oct. 1, 2004)], which is known to inhibit both raf kinase and VEGFR.

Until the present invention, there had not been a sensitive, quantitative and routine serum test to determine whether a cancer patient has a ras p21-driven tumor. The assays of this invention provide such sensitive and quantitative assays that can be used routinely, not only for serum, but for plasma, and other body fluids.

### SUMMARY OF THE INVENTION

The invention concerns the use of quantitative immunoassays according to claim 1 and 11 to measure serial changes in the levels of total ras p21 protein in human body fluids. Said serial changes are particularly useful as an indicator of the activation status of the ras signal transduction pathway, wherein "activation" is defined to include both upregulation and mutational activation. According to the theories underlying the methods of the invention, increasing total ras p21 protein (detected by serial changes in total ras p21 levels) is considered to be an indicator of an activated ras signal transduction pathway, signifying the presence of preneoplastic/neoplastic disease in a human subject. The immunoassays of the invention can be used clinically - diagnostically and/or prognostically, to detect precancer and/or cancer (preneoplastic/neoplastic disease) and as a therapeutic aid for patient therapy selection, monitoring the status of a preneoplastic/neoplastic disease in a patient, or monitoring how a patient with a preneoplastic/neoplastic disease is responding to a therapy. A preferred use for the immunoassays of the invention is to monitor the status of patients, and to make decisions about the optimal method for patient therapy.

Preferred samples in which to assay serial changes in total ras p21 by immmunoassay are samples of human body fluids, which can include, among other body fluids: blood, serum, plasma, urine, saliva, semen, breast exudate, cerebrospinal fluid, tears, sputum, mucous, lymph, cytosols, ascites, pleural effusions, amniotic fluid, bladder washes and bronchioalveolar lavages. Preferred body fluids to assay are blood, serum, and plasma, more preferably, serum or plasma. Particularly preferred body fluid samples include pretreatment samples and samples taken from a patient who has not responded to treatment.

In one aspect, the invention concerns diagnostic/prognostic methods to detect a preneoplastic/neoplastic disease associated with an activated ras pathway. One method of detecting a preneoplastic/neoplastic disease associated with an activated ras pathway in a human subject comprises the steps of:
(d) immunologically detecting and quantifying the average level of total ras p21 protein in samples of a body fluid taken from individuals of a control population;
(e) immunologically detecting and quantifying serial changes in total ras p21 protein levels in equivalent samples of body fluid taken from the subject over time; and
(f) comparing the levels of total ras p21 protein in the subject's samples to the average level of total ras p21 protein in the control samples;

wherein a level of total ras p21 protein in the subject's samples that is above the average level of total ras p21 protein in the control samples is indicative of an activated ras pathway and the presence of preneoplastic/neoplastic disease in the subject. The individuals of a control population of step (a) may be of either gender, or may be of the same gender as the subject.

Said method of detecting a preneoplastic/neoplastic disease associated with an activated ras pathway in a human subject may be further prognostic for said preneoplastic/neoplastic disease, wherein the levels of total ras p21 protein in the subject's samples relative to the average level of total ras p21 in the control samples are indicative of a better or poorer prognosis for said subject. Preferably, said prognosis is a clinical outcome selected from the group consisting of response rate (RR), complete response clinical benefit (CR), partial response clinical benefit (PR), stable disease clinical benefit (SD), time to progression (TTP), and time to death (TTD).

Said methods are in the form of a sandwich immunoassay, preferably a sandwich enzyme-linked immunosorbent assay (ELISA) or an equivalent assay. A preferred format is a sandwich ELISA. According to the invention the capture monoclonal antibody is Ras 17 which is secreted from hybridoma HB 10054 deposited at the American Type Culture Collection (ATCC), and the detector monoclonal antibody is Ras 10 which is secreted from hybridoma HB 9426 deposited at the ATCC, or Ras 10.2, which is secreted from a subclone of the Ras 10 hybridoma HB 9426. More preferably, the detector monoclonal antibody Ras 10 or Ras 10.2 is biotinylated.

Said methods can be diagnostic and/or prognostic for cancerous or precancerous diseases, wherein said disease is associated with an activated ras pathway. Exemplary of such precancerous or cancerous diseases are those selected from the group consisting of colorectal cancer, colon cancer, lung cancer, non-small-cell lung cancer, small-cell lung cancer, acute myelogenous leukemia, thyroid cancer, pancreatic cancer, bladder cancer, kidney cancer, melanoma, breast cancer, prostate cancer, ovarian cancer, cervical cancer, head-and-neck cancer, hepatocellular carcinoma and hematologic malignancies, and precancers leading to such cancers. Colon, colorectal, lung cancer and breast cancer, and precancers leading thereto, are considered particular targets for the diagnostic/prognostic assay methods that detect serial changes in total ras p21 in human body fluids.

A second aspect of the invention concerns the use of a quantitative immunoassay according to claim 1 to measure serial changes in the levels of total ras p21 protein in human body fluids, as a method of therapy selection for a human patient with a preneoplastic/neoplastic disease. One such method of therapy selection comprises the steps of:
(a) immunologically detecting and quantifying the average level of total ras p21 protein in samples of a body fluid taken from individuals of a control population;
(b)immunologically detecting and quantifying serial changes in total ras p21 protein levels in equivalent samples of body fluid taken from the patient over time;
(c) comparing the levels of total ras p21 protein in the patient's samples to the average level of total ras p21 protein in the control samples; and
(d) deciding whether to use conventional cancer therapy and/or ras-directed cancer therapy to treat the patient based upon the differences between the levels of total ras p21 protein in the patient's samples and the average level of total ras p21 protein in the control samples, and in view of the serial changes among the levels of total ras p21 protein in the patient's samples. For example, if a level of total ras p21 protein in a patient's sample is found to be above the average level of total ras p21 protein in the control samples, and if the serial changes in the total ras p21 protein levels in the patient's samples reflect levels that are trending above the average level of total ras p21 protein in the control samples, the conclusion could be drawn that the patient has a ras oncogene driven preneoplastic/ neoplastic disease, and the decision may be made to use ras-directed therapy to treat the patient, either alone or in conjunction with one or more other anticancer therapies.

Preferably, said ras-directed therapy is selected from the group consisting of farnesyltransferase inhibitors (FTI), tyrosine kinase inhibitors, bis-aryl ureas and antisense inhibitors of ras. More preferably, said ras-directed therapy is the bis aryl-urea sorafenib (BAY 43-9006).

Another aspect of the invention concerns the use of an immunoassay according to claim 11 as a method of monitoring the status of a preneoplastic/neoplastic disease in a patient, and/or monitoring how a patient with a preneoplastic/neoplastic disease is responding to a therapy, comprising immunologically detecting and quantifying serial changes in total ras p21 protein levels in samples of a body fluid taken from said patient over time; wherein increasing levels of total ras p21 protein over time indicate disease progression or a negative response to said therapy, and wherein decreasing levels of total ras p21 protein indicate disease remission or a positive response to said therapy. Said therapy may be conventional anticancer therapy, unconventional anticancer therapy, and/or a ras-directed therapy. A preferred body fluid sample, among other types of samples, may be a pretreatment sample, or a sample from a cancer patient who has not responded to treatment.

Still another aspect of this invention concerns the use of quantitative immunoassays according to claim 1 to detect changes in total ras p21 levels in combination with the levels of one or more other protein(s), in the methods of this invention as in the diagnostic/prognostic methods, in the methods of therapy selection for patients with a preneoplastic/neoplastic disease, in the methods of monitoring the status of a preneoplastic/neoplastic disease in a patient, and of monitoring how a patient with a preneoplastic/neoplastic disease is responding to a ras-directed or other therapy. Preferably, said other proteins that would be useful to quantitate along with ras p21 in the assays of this invention are inhibitors, oncoproteins, growth factor receptors, angiogenic factors, metastasis proteins, tumor markers and tumor suppressors. Preferably, said inhibitor is tissue-inhibitor of metalloproteinase-1 (TIMP-1), said oncoprotein is HER-2/neu, said growth factor receptors are selected from the group consisting of epidermal growth factor receptor (EGFR) and platelet derived growth factor receptor (PDGFR), said angiogenic factor is vascular endothelial growth factor (VEGF), said metastasis protein is urokinase-type plasminogen activator (uPA), said tumor marker is carcinoembryonic antigen (CEA), and said tumor suppressor is p53.

Most preferably, said other proteins are proteins that activate the ras pathway, such as TIMP-1 and HER-2/neu/c-erbB-2 ("HER-2/neu"). For example, increasing levels of total ras p21 and/or HER-2/neu may be indicative of a greater probability of early recurrence of a tumor or tumor metastasis. It would be advantageous to test cancer patients for serial changes in both total ras p21 and such other proteins, especially proteins that activate the ras pathway, to enlarge the clinical perspective, therapeutic resources and diagnostic/prognostic parameters to pick the optimal therapeutic combinations for the most promising treatment outcomes.

### REFERENCES

- Adjei, A.A., Journal of National Cancer Institute, 93(14): 1062-1074 (July 18, 2001)
- Agarwal et al., Oncogene, 20(20): 2527-2536 (May 3, 2001)
- Anderson et al., Mutat Res., 445(2):167-173 (Sep. 30, 1999)
- Anderson et al., Mutat. Res., 349 (1): 121-126 (Jan. 17, 1996)
- Anderson et al., Mutat. Res., 403(1-2): 229-235 (July 17, 1998)
- Anderson, D., Int. J. Hyg. Environ. Health, 204(1): 55-60 (Oct. 2001)
- Bos, J.L., Cancer Research. 49: 4682-4689 (1989)
- Bos, J.L., Mutation Research. 195: 255-271 (1988)
- Brandt-Rauf, P.W., J. Occup. Med., 33(9): 951-955 (Sep. 1991)
- Brtva et al., J Biol Chem., 270(17): 9809-9812 (April 28, 1995)
- Brunner et al., Cancer Research, 63: 5656-5668 (Sep. 15, 2003)
- Carney et al., "Monoclonal Antibodies for Detection of Normal and Oncogenic Ras p21," In Human Tumor Antigens and Specific Tumor Therapy, Alan R. Liss, Pub., pp. 53-62 (1989)
- Carney, W.P., The Cancer Journal, 4: 156-161 (1991)
- Carney, W.P., U.S. Patent No. 4,898,932 (Feb. 6, 1990)
- Carney, W.P., U.S. Patent No. 5,028,527 (July 2, 1991)
- Carney, W.P., U.S. Patent No. 5,081,230 (Jan. 14, 1992)
- Carney, W.P., U.S. Patent No. 5,084,380 (Jan. 28, 1992)
- Carney, W.P., U.S. Patent No. 5,112,737 (May 12, 1992)
- Carney, W.P., U.S. Patent No. 5,262,523 (Nov. 16, 1993)
- Carney, W.P., U.S. Patent No. 5,443,956 (August 22, 1995)
- Carney, W.P., U.S. Patent No. 6,200,764 B1 (March 13, 2001)
- Carney et al., Proc. Natl. Acad. Sci. (USA), 83: 7485-7489 (1986)
- Cebulska-Wasilewska A, et al., Mutat Res., 431(1): 123-131 (Dec. 16, 1999)
- Chang et al., Leukemia. 17(7): 1263-1293 (July 2003)
- Chie et al., Cancer Chemother Pharmocol, 45: 441-449 (2000)
- Choi et al., Oncogene, 23(1): 9-20 (2004)
- Collisson et al., Cancer Research. 63: 5669-5673 (Sep. 15, 2003)
- Cox and Der, Cancer Biol Ther., 1(6): 599:606 (Nov-Dec 2002)
- Dancey J. E., Curr Pharm Des., 8(25): 2259-2267 (2002)
- Dent et al., Oncogene, 22: 5885-5896 (2003)
- Eckert et al., Cancer Research, 64: 4585 - 4892 (July 1, 2004)
- Epelbaum et al., J Clin Lab Anal., 3(4): 209-214 (1989)
- Fischbach and Settleman, Cancer Research, 63: 4089-4094 (July 15, 2003)
- Fridman et al., J Biol Chem., 269(48): 30105-30108 (Dec. 2, 1994)
- Ghosh and Bell, J Bio Chem., 269(49): 30785-30788 (Dec. 9, 1994)
- Gibbs et al., Breast Cancer Res Treat., 38(1): 75-83 (1996)
- Hamer et al., Oncogene, 6(9): 1609-1615 (Sep. 1991)
- Holten-Andersen et al. , Int J Cancer, 113(2): 198-206 (Jan 10, 2005)
- Jin et al., British Journal of Cancer, 89, 185-191 (2003)
- Jones et al., Semin Radiat Oncol., 11(4): 328-337 (Oct. 2001)
- Karelia et al., Int. J. Biol. Markers. 16(2): 97-104 (April - June 2001)
- Kohl et al., J Cell Biochem (Suppl.), 22: 145-150 (1995)
- Lee C.S., Eur J Sura Oncol., 23(3): 233:237 (June 1997)
- Leevers et al., Nature. 369(6479): 411-414 (June 2, 1994)
- Lerner et al., J Biol Chem., 270(45): 26802-26806 (Nov. 10, 1995)
- Li et al., Development, 125(24): 4999-5008 (Dec. 1998)
- Liang et al., Mol Cancer Ther., 2(4): 353-360 (April 2003)
- Luo et al., J Occup Environ Med., 40(12):1053-1058 (Dec. 1998)
- Mallon et al., Anal Biochem, 294(1): 48-54 (July 1, 2001)
- Manzur et al., Cell Mol Neurobiol, 21(1): 39-52 (Feb. 2001)
- Marais et al., EMBO J., 14(1): 3136-3145 (July 3, 1995)
- Marshall, M., Mol Reprod Dev., 42(4): 493-499 (Dec. 1995)
- Meadows et al., Oncogene, 23(1): 192-200 (2004)
- Needleman et al., Pathology, 22(2): 77-81 (April 1990)
- Perera et al., Arch. Toxicol., 64(5): 401-406 (1990)
- Perera et al., Nature. 35: 256-258 (1992)
- Pitarque et al., Mutat Res., 440(2): 195-204 (April 6, 1999)
- Prior and Hancock, Journal of Cell Science, 114: 1603-1608 (2001)
- Pruitt and Der, Cancer Letters, 171: 1-10 (2002)
- Rundle et al., Cancer Lett., 185(1): 71-78 (Nov. 8, 2002)
- Schneider et al., Clin. Chem. Lab. Med., 38(4): 301-305 (April 2000)
- Singh et al., Cancer Res., 57(2): 253-258 (Jan. 15, 1997)
- Solanas and Escrich, Rev Esp Fisiol., 52(3):173-192 (Sep. 1996)
- Sommer et al., Oncogene, 22, 4266-4280 (2003)
- Sreelekha et al., J Esp Clin Cancer Res., 18 (3): 337-341 (Sep. 1999)
- Sun et al., Eur. J. Cancer. 27(12): 1646-1649 (1991)
- Takahashi et al., Clin Cancer Res., 1(10): 1071-1077 (Oct. 1995)
- Ulku et al., Mol Cancer Res.; 1(14): 1077-1088 (2003)
- Wang et al., J Cell Bio., 129(4): 1103-1114 (May 1995)
- Watson, D.M., Breast Cancer Res. Treat., 17(3):161-169 (Jan-Feb 1991)
- Weissfeld et al., Cancer Epidemiol Biomarkers Prev., 3(1): 57-62 (Jan-Feb 1994)
- Wilhelm et al., Cancer Research, 64: 7099-7109 (Oct. 1, 2004)
- Wittinghofer et al., FEBS Letters, 410: 63-67 (1997)
- Yan et al., Jour. Biol Chem., 273(37): 24052-24056 (Sep. 11, 1998)

### Abbreviations

The following abbreviations are used herein:
- ATCC -: American Type Culture Collection
- BSA -: bovine serum albumin
- °C -: degrees centigrade
- CEA -: carcinoembryonic antigen
- CV -: coefficient of variation
- EDTA -: ethylenediaminetetraacetate
- EGFR -: epidermal growth factor receptor
- ELISA -: enzyme-linked immunosorbent assay
- FTI -: farnesyltransferase inhibitor
- GDP -: guanosine diphosphate
- GEFS -: guanine-nucleotide exchange factors
- GTP -: guanosine triphosphate
- G protein -: guanyl nucleotide dependent protein
- HRP -: horseradish peroxidase
- kD -: kilodalton
- M -: molar
- Mab -: monoclonal antibody
- MAP -: mitogen-activated protein
- ml -: milliliter
- mM -: millimolar
- N -: normal or number (depending on context)
- ND -: not detected
- NED -: no evidence of disease
- Ng -: nanogram
- nm -: nanometer
- OD -: optical density
- pg -: proteoglycan
- PDGFR -: platelet derived growth factor receptor
- P. Resp -: patient's response
- Prog -: progressive disease
- Recur -: recurrence
- SD -: standard deviation
- Stab -: stable
- Stg -: stage
- TIMP-1 -: tissue inhibitor of metalloproteinase-1
- TMB -: tetramethylbenzidine
- µg -: microgram
- µl -: microliter
- Unk -: unknown
- uPA -: urokinase-type plasminogen activator
- VEGF -: vascular endothelial growth factor
- VEGFR -: vascular endothelial growth factor receptor

### BRIEF DESCRIPTION OF THE FIGURES

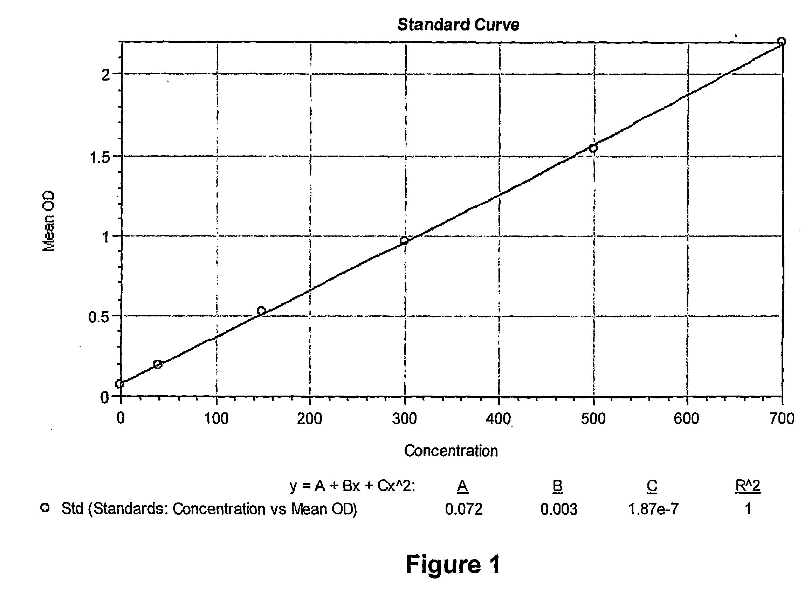

Figure 1 is an illustrative standard curve constructed by reacting a wild-type recombinant ras p21 standard at 6 different concentrations of 0, 40, 150, 300, 500 and 700 pg/ml in a preferred embodiment of the assays of this invention, a ras p21 ELISA, as described in detail in the Materials and Methods, and in Example 1, below.

### DETAILED DESCRIPTION

The present invention is directed to quantitative immunoassays that measure serial changes in the levels of total ras p21 protein in human body fluids, which assays are useful diagnostically/prognostically to detect or monitor a preneoplastic/neoplastic disease in a human, or to select a therapy for a patient with said preneoplastic/neoplastic disease, wherein said disease is associated with an activated ras pathway. As used herein, an "activated ras pathway" is defined as a ras pathway activated by either overexpression or mutation of ras p21 protein, and therefore encompasses upregulated and/or mutationally stimulated ras pathways. Further, as used herein, "total ras p21 protein" comprises both mutation-activated and normal (wild-type) p21 protein (including H-ras, K-ras, and N-ras proteins).

Exemplary of preneoplastic/neoplastic diseases associated with an activated ras pathway are the following, as well as precancers leading to the following: colorectal cancer, colon cancer, lung cancer, non-small-cell lung cancer, small-cell lung cancer, acute myelogenous leukemia, thyroid cancer, pancreatic cancer, bladder cancer, kidney cancer, melanoma, breast cancer, prostate cancer, ovarian cancer, cervical cancer, head-and-neck cancer, hepatocellular carcinoma and hematologic malignancies. In particular, the levels of total ras p21 protein, alone or in combination with levels of other proteins, particularly other oncoproteins, can be used to predict clinical outcome and/or as an aid in therapy selection.

The assays of this invention can be diagnostic and/or prognostic, i.e., diagnostic/prognostic. The term "diagnostic/ prognostic" is herein defined to encompass the following processes either individually or cumulatively depending upon the clinical context: determining the presence of disease, determining the nature of a disease, distinguishing one disease from another, forecasting as to the probable outcome of a disease state, determining the prospect as to recovery from a disease as indicated by the nature and symptoms of a case, monitoring the disease status of a patient, monitoring a patient for recurrence of disease, and/or determining the preferred therapeutic regimen for a patient. The diagnostic/prognostic methods of this invention are useful, for example, for screening populations for the presence of neoplastic or preneoplastic disease, determining the risk of developing neoplastic disease, diagnosing the presence of neoplastic and/or preneoplastic disease, monitoring the disease status of patients with neoplastic disease, and/or determining the prognosis for the course of neoplastic disease.

The present invention is useful for screening for the presence of a wide variety of preneoplastic/neoplastic diseases as indicated above. Such an assay can be used to detect tumors, monitor their growth, and help in the diagnosis and prognosis of disease. The assays can also be used to detect the presence of cancer metastasis, as well as confirm the absence of tumor tissue following cancer chemotherapy and/or radiation therapy. It can further be used to monitor cancer chemotherapy and tumor reappearance.

The methods include quantifying total ras p21 protein, if any, present in serial body fluid samples taken from a human subject diagnosed with, or suspected of having, a preneoplastic/neoplastic disease. The quantified total ras p21 protein levels are compared with the average levels in body fluid samples taken from individuals of a control population, wherein an above average level of total ras p21 protein is indicative of an activated ras pathway. As used herein, an "above average" level of total ras p21 protein indicates a level higher than two standard deviations (SD) above the mean level found in control samples. The individuals of the control population can be of either gender, or can be restricted to those who are of the same gender as the subject.

As used herein, "cancerous" and "neoplastic" have equivalent meanings, and "precancerous" and "preneoplastic" have equivalent meanings. The use of detection of gene expression products of oncogenes as diagnostic/prognostic indicators for preneoplastic/neoplastic diseases is considered conventional by those of skill in the art. However, the application of such approaches to measure quantitatively serial changes in ras levels in body fluids, particularly of circulating ras levels, to detect or monitor a preneoplastic/neoplastic disease, wherein said disease is associated with an activated ras pathway, is new.

### Serial Samples of Body Fluids

In a preferred embodiment of the invention, total ras p21 protein is quantitated in human body fluid samples drawn serially over time. Such body fluid samples can be blood, serum, plasma, urine, saliva, semen, breast exudate, cerebrospinal fluid, tears, sputum, mucous, lymph, cytosols, ascites, pleural effusions, amniotic fluid, bladder washes and bronchioalveolar lavages, among other body fluid samples. Preferred body fluids, for example, include serum, or plasma samples treated with heparin, citrate or EDTA, among other body fluid samples, and can be fresh or frozen. Such body fluid specimens can be taken pretreatment, during treatment, or post-treatment, or can be taken from a patient who is not responding to therapy. As used herein, "serial changes over time" or "serial samples" denotes sequential testing of samples taken over time periods which would be considered relevant for the subject, depending on the context and the circumstances. For example, for cancer patient screening, serial samples might be drawn upon a patient's initial visit, after diagnosis, pre-surgery and/or post-surgery; whereas for population screening for a preneoplastic disease, serial samples might be drawn on a yearly basis.

### Immunoassays

An exemplary and preferred immunoassay according to the methods of the invention is a sandwich ELISA described below in the Materials and Methods section, and was used to obtain the data for Examples 1 through 8. It can be appreciated that alternate methods, in addition to those disclosed herein, can be used to quantify total ras p21 protein in human body fluids. Other preferred sandwich assays could be used with other visualizing means, such as luminescent labels. Other labels are detailed below under the subsection labeled Labels.

Many formats can be adapted for use with the methods of the present invention. The detection and quantitation of total ras p21 protein in human body fluids can be performed, for example, by enzyme-linked immunosorbent assays, radioimmunoassays, dual antibody sandwich assays, agglutination assays, fluorescent immunoassays, immunoelectron and scanning microscopy using immunogold, among other assays commonly known in the art. The quantitation of total ras p21 protein in such assays can be adapted by conventional methods known in the art. In preferred embodiments, serial changes in circulating total ras p21 protein levels, or such levels in other body fluids, are detected and quantified by a sandwich assay in which the capture antibody has been immobilized, using conventional techniques, on the surface of the support.

Suitable supports used in assays include among other supports, synthetic polymer supports, such as polypropylene, polystyrene, substituted polystyrene, polyacrylamides (such as polyamides and polyvinylchloride), glass beads, agarose, and nitrocellulose, among other supports.

### Exemplary Immunoassay

An exemplary and preferred ELISA sandwich immunoassay is described in the Materials and Methods section and in Examples 1-8. That exemplary ELISA uses Mab Ras 17 as the capture antibody and biotinylated Mab 10.2 (produced by a subclone of hybridoma Ras 10) as the detector antibody. The capture Mab Ras 17 is immobilized on microtiter plate wells; diluted human serum/plasma samples or ras p21 standards (recombinant wild-type H-ras protein) are incubated for three hours at 37°C in the wells to allow binding of total ras p21 antigen by Mab 17. After washing of wells, the immobilized ras p21 antigen is exposed to the biotinylated detector antibody Mab Ras 10.2 for 30 minutes at room temperature (20-27°C), after which the wells are again washed. A streptavidinhorseradish peroxidase conjugate is then added (30 minutes at room temperature). After a final wash, TMB Blue Substrate is added to the wells (and incubated for 30 minutes at room temperature) to detect bound peroxidase activity. The reaction is stopped by the addition of 2.5N sulfuric acid, and the absorbance is measured at 450 nm. Correlating the absorbance values of samples with the ras standards allows the determination of a quantitative value of total ras p21 in pg/ml of serum or plasma.

### Combination Assays: Levels of Ras and Other Protein(s)

It can be appreciated that other proteins, such as inhibitors, oncoproteins, growth factor receptors, angiogenic factors, metastasis proteins, tumor markers, and tumor suppressors, particularly those associated with the ras pathway, may be suitable for detection and quantitation along with ras p21, depending on the subject preneoplastic/neoplastic disease. Exemplary and preferred other proteins suitable for testing along with ras p21 include tissue inhibitor of metalloproteinase-1 (TIMP-1), HER-2/neu, epidermal growth factor receptor (EGFR), platelet derived growth factor receptor (PDGFR), vascular endothelial growth factor (VEGF), urokinase-type plasminogen activator (uPA), carcinoembryonic antigen (CEA), and p53. Assays to detect preferred other proteins suitable for the methods of the invention are known to one of skill in the art. For example, immunoassays for the quantitation of HER-2/neu and TIMP-1 in human body fluids are commercially available, such as the Oncogene Science TIMP-1 ELISA [Oncogene Science, Cambridge, MA (USA), www.oncogene.com], which can determine ng/ml values of TIMP-1 levels in human serum or plasma. As described in Example 8, serial colorectal cancer serum samples and normal serum samples were assayed for both total ras p21 and TIMP-1 levels.

### Prognosis

Monitoring the levels of ras p21 and/or other proteins can be indicative of clinical outcomes for preneoplastic/neoplastic diseases. A preferred method of evaluating a clinical outcome is one based on response rate (RR), clinical benefit [including complete response (CR), partial response (PR), and stable disease (SD)], time to progression (TTP), and time to death (TTD). Other methods of evaluating prognosis are known in the art and can be used.

### Antibodies

Antibodies used in the immunoassay of the invention are the Ras 17 and Ras 10 antibodies, or antibodies produced by subclones of the Ras 17 and Ras 10 hybridomas (such as the Ras 10.2 Mab from a subclone of the parent Ras 10 hybridoma), so long as they exhibit the desired biological activity.

"Antibody fragments" comprise a portion of a full length antibody, generally the antigen binding or variable domain thereof. Examples of antibody fragments include Fab, Fab', F(ab')₂, and Fv fragments, diabodies, linear antibodies, and single-chain antibody molecules, as well as multispecific antibodies, including bispecific antibodies, formed from antibody fragments.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature 256:495 (1975), or may be made by recombinant DNA methods (see, e.g., U.S. Pat. No. 4,816,567). The "monoclonal antibodies" may also be isolated from phage antibody libraries using the techniques described in Clackson et al., Nature. 352: 624-628 (1991) and Marks et al., J. Mol. Biol., 222: 581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity [U.S. Pat. No. 4,816,567; and Morrison et al., Proc. Natl. Acad. Sci., USA, 81: 6851-6855 (1984)].

"Humanized" forms of non-human (e.g., murine) antibodies are chimeric antibodies which contain minimal sequence derived from non-human immunoglobulin. For the most part, humanized antibodies are human immunoglobulins (recipient antibody) in which hypervariable region residues of the recipient are replaced by hypervariable region residues from a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues which are not found in the recipient antibody or in the donor antibody. Such modifications are made to further refine antibody performance. In general, the humanized antibody will comprise substantially all of at least one, and typically two, variable domains, in which all or substantially all of the hypervariable regions correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, see Jones et al., Nature. 321:522-525 (1986); Reichmann et al., Nature, 332: 323-329 (1988); and Presta, Curr. Op. Struct. Biol., 2: 593-596 (1992).

"Single-chain Fv" or "sFv" antibody fragments comprise the V_{H} and V_{L} domains of antibody, wherein these domains are present in a single polypeptide chain. Generally, the Fv polypeptide further comprises a polypeptide linker between the V_{H} and V_{L} domains which enables the sFv to form the desired structure for antigen binding. For a review of sFv see Pluckthun in The Pharmacology of Monoclonal Antibodies, Vol. 113, Rosenburg and Moore eds. Springer-Verlag, New York, pp. 269-315 (1994).

The term "diabodies" refers to small antibody fragments with two antigen-binding sites, which fragments comprise a heavy chain variable domain (V_{H}) connected to a light chain variable domain (V_{L}) in the same polypeptide chain (V_{H}-V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci., USA, 90: 6444-6448 (1993).

The expression "linear antibodies" refers to the antibodies described in Zapata et al., Protein Eng., 8(10): 1057-1062 (1995). Briefly, such antibodies comprise a pair of tandem Fd segments (V_{H}-C_{H}1-V_{H}-C_{H}1) which form a pair of antigen binding regions. Linear antibodies can be bispecific or monospecific.

Representative monoclonal antibodies useful according to this invention include Mabs Ras 17 and Ras 10 described in earlier Carney et al. patents [US Pat. No. 5,081,230; US Pat. No 5,443,956; US Pat. No.6,200,764]. Monoclonal antibodies useful according to this invention serve to identify total ras p21 proteins in various laboratory prognostic tests, for example, in clinical samples.

General texts describing additional molecular biological techniques useful herein, including the preparation of antibodies include Berger and Kimmel, Guide to Molecular Cloning Techniques, Methods in Enzymology, Vol. 152, Academic Press, Inc., Sambrook et al., Molecular Cloning: A Laboratory Manual, (Second Edition, Cold Spring Harbor Laboratory Press; Cold Spring Harbor, N.Y.; 1989) Vol. 1-3; Current Protocols in Molecular Biology, F. M. Ausabel et al. [Eds.], Current Protocols, a joint venture between Green Publishing Associates, Inc. and John Wiley & Sons, Inc. (supplemented through 2000), Harlow et al., Monoclonal Antibodies: A Laboratory Manual, Cold Spring Harbor Laboratory Press (1988), Paul [Ed.]; Fundamental Immunology, Lippincott Williams & Wilkins (1998), and Harlow et al., Using Antibodies: A Laboratory Manual, Cold Spring Harbor Laboratory Press (1998).

### Labels

The antibodies useful according to this invention to identify total ras p21 proteins can be labeled in any conventional manner. A preferred label, according to this invention is horseradish peroxidase, and a preferred method of labeling the antibodies is by using biotin-strepavidin complexes.

As appropriate, antibodies used in the immunoassays of this invention that are used as tracers may be labeled in any manner, directly or indirectly, that results in a signal that is visible or can be rendered visible. Detectable marker substances include radionuclides, such as ³H, ¹²⁵I, and ¹³¹I; fluorescers, such as, fluorescein isothiocyanate and other fluorochromes, phycobiliproteins, phycoerythin, rare earth chelates, Texas red, dansyl and rhodamine; colorimetric reagents (chromogens); electron-opaque materials, such as colloidal gold; bioluminescers; chemiluminescers; dyes; enzymes, such as, horseradish peroxidase, alkaline phosphatases, glucose oxidase, glucose-6-phosphate dehydrogenase, acetylcholinesterase, alpha -, beta-galactosidase, among others; coenzymes; enzyme substrates; enzyme cofactors; enzyme inhibitors; enzyme subunits; metal ions; free radicals; or any other immunologically active or inert substance which provides a means of detecting or measuring the presence or amount of immunocomplex formed. Exemplary of enzyme substrate combinations are horseradish peroxidase and tetramethyl benzidine (TMB), and alkaline phosphatases and paranitrophenyl phosphate (pNPP).

Another preferred detection, or detection and quantitation systems according to this invention produce luminescent signals, bioluminescent (BL) or chemiluminescent (CL). In chemiluminescent (CL) or bioluminescent (BL) assays, the intensity or the total light emission is measured and related to the concentration of the unknown analyte. Light can be measured quantitatively using a luminometer (photomultiplier tube as the detector) or charge-coupled device, or qualitatively by means of photographic or X-ray film. The main advantages of using such assays are their simplicity and analytical sensitivity, enabling the detection and/or quantitation of very small amounts of analyte.

Exemplary luminescent labels are acridinium esters, acridinium sulfonyl carboxamides, luminol, umbelliferone, isoluminol derivatives, photoproteins, such as aequorin, and luciferases from fireflies, marine bacteria, Vargulla and Renilla. Luminol can be used optionally with an enhancer molecule, preferably selected from the group consisting of 4-iodophenol or 4-hydroxy-cinnamic acid. Acridinium esters are one of the preferred types of CL labels according to this invention. Typically, a CL signal is generated by treatment with an oxidant under basic conditions.

Also preferred luminescent detection systems are those wherein the signal (detectable marker) is produced by an enzymatic reaction upon a substrate. CL and BL detection schemes have been developed for assaying alkaline phosphatases (AP), glucose oxidase, glucose 6-phosphate dehydrogenase, horseradish peroxidase (HRP), and xanthine-oxidase labels, among others. AP and HRP are two preferred enzyme labels which can be quantitated by a range of CL and BL reactions. For example, AP can be used with a substrate, such as an adamantyl 1,2-dioxetane aryl phosphate substrate (e.g. AMPPD or CSPD; [Kricka, L.J., "Chemiluminescence and Bioluminescence, Analysis by," at p. 167, Molecular Biology and Biotechnology: A Comprehensive Desk Reference (ed. R.A. Meyers) (VCH Publishers; N.Y., N.Y.; 1995)]; preferably a disodium salt of 4-methoxy-4-(3-phosphatephenyl) spiro [1,2-dioxetane-3,2'-adamantane], with or without an enhancer molecule, preferably, 1-(trioctylphosphonium methyl)-4- (tributylphosphonium methyl) benzene diochloride. HRP is preferably used with substrates, such as, 2',3',6'- trifluorophenyl 3-methoxy-10-methylacridan-9-carboxylate.

CL and BL reactions can be adapted for analysis not only of enzymes, but also of other substrates, cofactors, inhibitors, metal ions and the like. For example, luminol, firefly luciferase, and marine bacterial luciferase reactions are indicator reactions for the production or consumption of peroxide, ATP, and NADPH, respectively. They can be coupled to other reactions involving oxidases, kinases, and dehydrogenases, and can be used to measure any component of the coupled reaction (enzyme, substrate, cofactor).

The detectable marker may be directly or indirectly linked to an antibody used in an assay of this invention. Exemplary of an indirect linkage of the detectable label is the use of a binding pair between an antibody and a marker or the use of a signal amplification system.

Exemplary of binding pairs that can be used to link antibodies of assays of this invention to detectable markers are biotin/avidin, streptavidin, or anti-biotin; avidin/anti-avidin; thyroxine/thyroxine-binding globulin; antigen/antibody; antibody/ anti-antibody; carbohydrate/lectins; hapten/anti-hapten antibody; dyes and hydrophobic molecules/hydrophobic protein binding sites; enzyme inhibitor, coenzyme or cofactor/enzyme; polynucleic acid/homologous polynucleic acid sequence; fluorescein/anti- fluorescein; dinitrophenol/anti-dinitrophenol; vitamin B12/intrinsic factor; cortisone, cortisol/cortisol binding protein; and ligands for specific receptor protein/membrane associated specific receptor proteins. Preferred binding pairs according to this invention are biotin/avidin or streptavidin, more preferably biotin/ streptavidin.

Various means for linking labels directly or indirectly to antibodies are known in the art. For example, labels may be bound either covalently or non-covalently. Exemplary antibody conjugation methods are described in: Avarmeas et al., Scan. J. Immunol., 8 (Suppl. 7): 7 (1978); Bayer et al., Meth. Enzymol., 62: 308 (1979); Chandler et al., J. Immunol. Meth., 53: 187 (1982); Ekeke and Abuknesha, J. Steroid Biochem., 11: 1579 (1979); Engvall and Perlmann, J. Immunol., 109: 129 (1972); Geoghegan et al., Immunol. Comm., 7: 1 (1978); and Wilson and Nakane, Immunofluorescence and Related Techniques, p. 215 [Elsevier/North Holland Biomedical Press; Amsterdam (1978)].

Depending upon the nature of the label, various techniques can be employed for detecting and quantitating the label. For fluorescers, a large number of fluorometers are available. For chemiluminescers, luminometers or films are available. With enzymes, a fluorescent, chemiluminescent, or colored product can be determined or measured fluorometrically, luminometrically, spectrophotometrically or visually.

Various types of chemiluminescent compounds having an acridinium, benzacridinium, or acridan type of heterocyclic ring systems are preferred labels. Acridinium and benzacridinium esters are currently the more preferred chemiluminescent compounds, with preferred acridinium esters including those compounds having heterocyclic rings or ring systems that contain the heteroatom in a positive oxidation state including such ring systems as acridinium, benz[a]acridinium, benz[b]acridinium, benz[c]acridinium, a benzimidazole cation, quinolinium, isoquinolinium, quinolizinium, a cyclic substituted quinolinium, phenanthridinium, and quinoxalinium, as are well-known in the art.

The tracer may be prepared by attaching to the selected antibody either directly or indirectly a reactive functional group present on the acridinium or benzacridinium ester, as is well known to those skilled in the art, e.g. Weeks et al., Clinical Chemistry, 29(8), 1474-1479, (1983). Particularly preferred compounds are acridinium and benzacridinium esters with an aryl ring leaving group and the reactive functional group present in either the para or the meta position of the aryl ring. See, U.S. Patent No. 4,745,181 and WO 94/21823.]

### Ras-Directed Therapies

As used herein, "ras-directed therapies" include any therapies that are targeted to the ras pathway, including inhibition of ras protein expression (e.g., antisense oligonucleotides), prevention of membrane localization essential for ras activation (ras farnesylation inhibitors), or inhibition of downstream effectors of ras (e.g., Raf serine/threonine kinases). Preferred ras-directed therapies include farnesyltransferase inhibitors (FTIs), tyrosine kinase inhibitors, or bis-aryl ureas. Exemplary FTls are BMS-214662 [an imidazole-containing nonpeptidomimetic FTI; Cortes et al., J. Clin. Oncol. 23 (12): 2805-2812 (April 20, 2005); Bristol-Myers Squibb, Princeton, NJ (USA)], FTI-2153 and FTI-277 [EMD Biosciences, www.emdbiosciences.com (Cat. No. 3444555); Lerner et al. (1995); Sun et al., Cancer Res., 59: 4919-4926 (1999)], R115777 [methylquinolone; Janssen Research Foundation, Titusville, NJ (USA)], and Sch66336 [Sarasar® or Lonafarnib®; a benzocycloheptapyridyl FTI; Taveras et al., Curr. Topics Med. Chem., 3(10): 1103-1114 (June 2003); Schering Plough, Kenilworth, NJ (USA)].

An exemplary tyrosine kinase inhibitor is AZD3049 [prenyl transferase inhibitor; AstraZeneca, www.astrazeneca.com]. An exemplary antisense inhibitor of ras is ISIS 2503 [ISIS Pharmaceuticals, Inc., Carlsbad, CA (USA)]. [See also, for example, hftp://www.webtie.org/sots/html/LungAgents.htm, for other ras-directed therapies targeted to ras or at relevant effectors downstream from ras.]

A preferred ras-directed therapy according to the invention is the bis-aryl urea sorafenib (BAY 43-9006) [Onyx Pharmaceuticals, Richmond, CA (USA), and Bayer Corporation, West Haven, CT (USA); Lyons et al., Endocrine-Related Cancer, 8:219-225 (2001) and Wilhelm et al. (2004)], a small molecule which inhibits the enzyme Raf kinase. Sorafenib targets both the RAF/MEK/ERK [MEK: MAP/ERK kinase; ERK: extracellular signal related kinase] signaling pathway to inhibit cell proliferation, and the VEGFR-2/PDGFR-β signaling pathway to inhibit tumor angiogenesis. Sorafenib then would be a particularly preferred anticancer therapy for a patient found to have an activated ras pathway and an activated VEGFR-2/PDGFR-β signaling pathway.

### EXAMPLES

The following examples are for purposes of illustration only and are not meant to limit the invention in any way.

### Materials and Methods

### Solid Phase Sandwich Microtiter ELISA for Human Serum and Plasma

### Sample Preparation

Suitable samples for analysis by the ras p21 ELISA include human plasma treated with heparin, citrate, or EDTA, and human serum. Due to possible interfering factors, special care must be taken in the preparation and assay of human serum and plasma. Any flocculant material should be removed from samples by microcentrifugation prior to dilution. The initial concentration of the serum or plasma specimen to be examined should not exceed a concentration of 50% (a 1:2 dilution of specimen in sample diluent). For example, 0.150 ml of sample may be diluted into 0.150 ml of sample diluent, and 100 µl added to the microplate wells.

### Exemplary Assay Procedure

Purified Ras 17 Mabs [secreted from the Ras 17 hybridoma deposited at the ATCC under HB 10054] are diluted to 1 µg/ml in coating buffer (0.03 M sodium carbonate anhydrous and 0.07 M sodium bicarbonate, pH 9.6) and are adsorbed to wells (at 100 µl per well) of 96-well microplates [Nunc MaxiSorp™ F8 Immuno Module, Part#1001; Nalge Nunc International Corp., Naperville, IL (USA)]. Adsorption is carried out overnight (at least 12 hours) at room temperature (20°-27°C). At the end of the incubation period, blocking solution is added to the wells (0.002M sodium phosphate monobasic, 0.008M sodium phosphate dibasic heptahydrate, 0.15M sodium chloride, 0.29M beta D-lactose and 2% BSA reagent grade; pH 7.45) and aspirated using an Oyster Bay Microplate Coater (Model MPCS-3) [Oyster Bay Pump Works, Hicksville, N.Y. (USA)]. Coated microplates are dried using a LyoStar® Freeze-Dryer [FTS Systems, Stone Ridge, N.Y. (USA)] at 22°C and stored at 2-8°C.

Human serum samples and ras p21 standards [Invitrogen Corporation, Catalog # P2138; Carlsbad, CA (USA)] are diluted serially with sample diluent [4% BSA Protease Free (Serologicals Product #82-045; www.serologicals.com), 10 µg/ml Purified Mouse IgG (Scantibody Laboratories Product #3BM222; Santel, CA, USA) and 0.09% sodium azide]. Plasma samples treated with heparin, citrate or EDTA may be used instead of serum. Diluted samples are then added to microplate wells (100 µl per well) and the sealed plates incubated for 3 hours at 37°C. Each well is washed six times with 300 µL per well of platewash, pH 7.4 [0.137M sodium chloride, 2.7mM potassium chloride, 1.45 mM potassium phosphate monobasic, 0.08 M sodium phosphate dibasic heptahydrate, and 0.05% Tween 20]. To each well is then added 100 µl of biotinylated Ras 10.2 Mabs [whose parent hybridoma Ras 10 is on deposit at the ATCC under HB 9426] diluted in detector diluent [0.4% casein, 1.7mM sodium phosphate monobasic, 8.1 mM sodium phosphate dibasic heptahydrate, 0.15M sodium chloride, and 0.1 % sodium azide, pH 7.15±0.1] (final Mab concentration 0.5-3.5 µg/ml; optimal concentration determined by titration and functional testing of detector Mab).

The plates are incubated for 30 minutes at room temperature. Each well is washed six times as above, and 100 µl of Strepavidin-HRP [Zymed Laboratories, Inc., Catalog # 43-8323; www.zymed.com] diluted to 1 µg/mL or less in conjugate diluent [1.7mM sodium phosphate monobasic, 8.1 mM sodium phosphate dibasic heptahydrate, 0.145M sodium chloride, 0.1% 2-chloroacetamide, 1% BSA Protease Free, and 0.05% Tween 20; pH 7.3] is added to each well. The plates are incubated at room temperature for 30 minutes. Each well is again washed six times as above, and 100 µl of a color producing substrate solution [TMB Blue Substrate, DakoCytomation, Catalog # S1601; www.dakocytomation.com] is added to each well to detect bound peroxidase activity. The plates are incubated 30 minutes at room temperature, and the reaction is stopped by the addition of 100 µl 2.5N sulfuric acid. The absorbance is measured at 450 nm using a Molecular Devices spectrophotometric plate reader (Model #Vmax; www.moleculardevices.com) and analyzed using a Softmax Pro^{™} (Molecular Devices Corporation; www.moleculardevices.com) software program.

### Human Serum and Plasma Samples

Frozen plasma and serum samples from normal humans and cancer patients were obtained from Bioclinical Partners, Inc. [Franklin, MA (USA); now Zeptometrix (www.zeptometrix.com)].

### Example 1

### Ras Standard Curve

Quantitative analyses were made by constructing a standard curve using Ha ras p21 [recombinant wild-type ras p21 from Invitrogen Corporation, Catalog # P2138]. Figure 1 presents the standard curve which was constructed by reacting various concentrations of the Ha-ras p21 protein with immobilized anti-p21 pan reactive monoclonal antibody Ras 17 and detecting captured ras p21 protein using biotinylated anti-total p21 monoclonal antibody Ras 10.2. The immunoassay procedure was as described above in Materials and Methods.

### Example 2

### Normal Human Serum

Microplate wells were coated with anti-total ras p21 capture antibody (Mab Ras 17), as described above. A total of 82 normal human sera (42 female, 40 male; obtained from Bioclinical Partners, Inc.) were individually tested in the coated microplates by ELISA, and ras p21 present in the sera was detected using biotinylated Ras 10.2 monoclonal antibody. The absorbance index at 450 nm was used to determine the level of total ras p21 in serum samples. The results show that, in general, higher levels of total ras p21 protein were found in normal females than in normal males (average of 122.4 pg/ml in females vs. 33.1 pg/ml in males).

**Table 1: Single Point Serum samples from Normal Humans**

| **Normal Female** | | **Normal Male** | |
|---|---|---|---|
| **Serum Samples** | | **Serum Samples** | |
| **Sample** | **Ras p21 (pg/mL)** | **Sample** | **Ras p21 (pg/mL)** |
| 3666 | 204.4 | 3552 | 54.3 |
| 3668 | 148.4 | 3553 | 54.5 |
| 3669 | 119.7 | 3554 | 73.9 |
| 3670 | 137.5 | 3555 | 40.6 |
| 3671 | 235.6 | 3556 | 36.2 |
| 3672 | 134.8 | 3557 | 46.3 |
| 3673 | 223.0 | 3558 | 41.9 |
| 3674 | 146.8 | 3559 | 37.0 |
| 3675 | 91.6 | 3560 | 2.2 |
| 3676 | 75.2 | 3561 | 7.0 |
| 3677 | 100.7 | 3562 | 5.0 |
| 3678 | 337.9 | 3563 | 5.6 |
| 3679 | 99.7 | 3564 | 0.8 |
| 3680 | 303.8 | 3565 | 1.5 |
| 3681 | 136.5 | 3566 | 5.4 |
| 3682 | 76.1 | 3567 | 2.0 |
| 3683 | 64.6 | 3568 | 4.6 |
| 3684 | 80.6 | 3569 | 6.4 |
| 3685 | 168.1 | 3570 | 4.0 |
| 3686 | 85.9 | 3571 | 10.3 |
| 3687 | 120.2 | 3572 | 1.1 |
| 3688 | 138.9 | 3573 | 98.8 |
| 3689 | 127.0 | 3574 | 3.7 |
| 3690 | 57.6 | 3575 | 4.8 |
| 3691 | 68.0 | 3576 | 35.9 |
| 3692 | 49.4 | 3577 | 37.6 |
| 3693 | 183.1 | 3578 | 5.4 |
| 3694 | 50.1 | 3579 | 10.8 |
| 3695 | 62.5 | 3580 | 5.0 |
| 3696 | 49.2 | 3581 | 5.1 |
| 3697 | 48.6 | 3582 | 4.7 |
| 3698 | 59.1 | 3583 | 3.5 |
| 3699 | 68.8 | 3584 | 4.4 |
| 3700 | 63.6 | 3585 | 8.9 |
| 3701 | 395.1 | 3586 | 10.6 |
| 3702 | 49.7 | 3587 | 16.5 |
| 3703 | 47.6 | 3588 | 6.6 |
| 3704 | 60.8 | 3589 | 19.3 |
| 3705 | 56.4 | 3590 | 453.1 |
| 3706 | 62.8 | 3591 | 150.5 |
| 3707 | 228.0 | | |

**Table 1 Summary**

| RAS Levels in Normal Human Serum (pg/ml) | | | | | |
|---|---|---|---|---|---|
| Gender | N | Range | Mean | Mean + 2SD | 95% fall below: |
| Male | 40 | ND - 453.1 | 33.1 | 182.5 | 101.4 |
| Female | 41 | ND - 395.1 | 122.4 | 289.7 | 303.8 |

### Example 3

### Plasma vs. Serum

Sixteen matched normal human serum and plasma samples were assayed for total ras p21 protein levels by ELISA, as described above in Materials and Methods. Plasma levels averaged approximately 24% higher than serum levels, as demonstrated in Table 2 below.

**Table 2**

| **Matched Normal Human Serum /Plasma (pg/ml)** | | | |
|---|---|---|---|
| **Sample#** | **Plasma** | **Serum** | **% Difference** |
| 1602 | 166.659 | 146.964 | 11.8 |
| 1603 | 131.963 | 121.974 | 7.6 |
| 1604 | 111.289 | 87.124 | 21.7 |
| 1605 | 116.629 | 87.124 | 25.3 |
| 1606 | 171.328 | 102.398 | 40.2 |
| 1607 | 167.74 | 102.398 | 39.0 |
| 1608 | 178.863 | 106.667 | 40.4 |
| 1609 | 477.954 | 147.325 | 69.2 |
| 1610 | 147.328 | 140.53 | 4.6 |
| 1611 | 149.834 | 85.351 | 43.0 |
| 1612 | 154.124 | 127.678 | 17.2 |
| 1613 | 73.658 | 79.324 | -7.7 |
| 1614 | 116.634 | 110.937 | 4.9 |
| 1615 | 125.182 | 117.341 | 6.3 |
| 4029 | 256.486 | 160.924 | 37.3 |
| 4031 | 132.319 | 98.486 | 25.6 |
| **Mean** | **167.37** | **113.91** | **24.14** |
| **SD** | **91.9** | **25.0** | **19.8** |

### Example 4

### Assay Characteristics

The sensitivity, specificity, precision and parallelism of the ELISA assay for total ras p21 in human serum or plasma was characterized (in all instances, using Mab Ras 17 as the capture antibody and Mab Ras 10.2 as the detector antibody).

### 1. Analytical Sensitivity

A minimal detectable concentration of analyte was determined by repeated measurement of a zero dose sample (Standard Level 1) and calculation of mean + 2 standard deviations. The ras ELISA assay will detect 4.6 picograms/ml of total ras p21 protein.

### 2. Specificity

Cross reactivity: Rab 3A, Rac-1, and Ran are other members of the G-protein family. The Oncogene Science RAS p21 ELISA was challenged with high levels of each analyte. Only Rab-3A showed any cross-reactivity at a minimal 1.22%.

### 3. Precision

Inter-Assay Precision: The reproducibility of the ELISA test for determining total ras p21 protein in human samples was confirmed by repeated testing of the same sera on different occasions (Table 3). Normal human serum and plasma were spiked with recombinant wild-type ras p21 [Invitrogen] at three different concentrations and tested in 8 assays with 8 replicates per test point. Between-assay variability was less than or equal to 10.0%.

**Table 3**

| Inter-Assay Precision | | | |
|---|---|---|---|
| | High | Medium | Low |
| n | 64 | 64 | 64 |
| Serum Mean (pg/ml) | 472.9 | 320.0 | 115.7 |
| Plasma Mean (pg/ml) | 371.3 | 167.8 | 71.5 |
| Serum Inter-Assay % CV | 7.1 | 8.6 | 10.0 |
| Plasma Inter-Assay % CV | 8.7 | 7.5 | 8.0 |

Intra-Assay Precision: Normal human serum and plasma were spiked with ras p21 protein at three different concentrations and tested in 8 assays with 8 replicates per test point. Mean intra-assay variability was less than 7% (Table 4).

**Table 4**

| Intra-Assay Precision | | | |
|---|---|---|---|
| | High | Medium | Low |
| n | 64 | 64 | 64 |
| Serum Mean (pg/ml) | 472.9 | 320.0 | 115.7 |
| Plasma Mean (pg/ml) | 371.3 | 167.8 | 71.5 |
| Serum Intra-Assay % CV | 5.8 | 6.6 | 6.9 |
| Plasma Intra-Assay % CV | 6.5 | 6.0 | 6.6 |

### 4. Parallelism

Spiked serum and plasma samples were initially diluted 1:2, subsequent 2 fold dilutions were made, and all dilutions were tested in the ras p21 ELISA. Recoveries (pg/ml) for each dilution were calculated by correcting with the appropriate dilution factor. Results show that spiked plasma and serum samples diluted in sample diluent result in accurate recovery of ras p21 protein (Table 5).

**Table 5**

| **Parallelism** | | | | |
|---|---|---|---|---|
| | **Plasma (EDTA)** | | **Serum** | |
| **Dilution** | **OD** | **pg/ml** | **OD** | **pg/ml** |
| 1:2 | 1.516 | 485.909 | 1.707 | 549.869 |
| 1:4 | 0.869 | 538.608 | 0.937 | 554.674 |
| 1:8 | 0.496 | 577.219 | 0.470 | 564.503 |
| 1:16 | 0.264 | 534.753 | 0.237 | 524.047 |
| | | | | |

| | **Plasma (Citrate)** | | **Plasma (Heparin)** | |
|---|---|---|---|---|
| **Dilution** | **OD** | **pg/ml** | **OD** | **pg/ml** |
| 1:2 | 1.728 | 556.734 | 1.689 | 543.841 |
| 1:4 | 0.932 | 580.448 | 0.883 | 547.645 |
| 1:8 | 0.475 | 549.780 | 0.455 | 522.341 |
| 1:16 | 0.279 | 573.564 | 0.262 | 529.400 |

### Example 5

### Ras Levels in Serial Samples from Normal Serum

Serial serum samples from fifteen normal humans (thirteen males, two females) were assayed by ELISA for total ras p21 levels as described above under Materials and Methods. In general, the samples were drawn at least three weeks apart.

**Table 6**

| Serial Samples from Normal individuals (Ras Levels) | | | |
|---|---|---|---|
| Sample | Ras p21 (pg/mL) | Date of Draw | Gender |
| **3651-1** | 159.0 | 5/20/2001 | Male |
| **3651-2** | 221.1 | 6/12/2001 | |
| **3651-3** | 141.3 | 7/14/2001 | |
| | | | |
| **3652-1** | 50.6 | 3/20/2001 | Male |
| **3652-2** | 56.0 | 5/8/2001 | |
| **3652-3** | 58.8 | 6/19/2001 | |
| | | | |
| **3653-1** | 246.7 | 1/12/2001 | Male |
| **3653-2** | 560.0 | 4/4/2001 | |
| **3653-3** | 244.4 | 9/17/2001 | |
| | | | |
| **3654-1** | 113.9 | 1/5/2001 | Male |
| **3654-2** | 127.9 | 1/31/2001 | |
| **3654-3** | 170.2 | 2/21/2001 | |
| **3654-4** | 126.7 | 9/6/2001 | |
| | | | |
| **3655-1** | 101.0 | 3/22/2001 | Male |
| **3655-2** | 99.6 | 5/9/2001 | |
| **3655-3** | 132.3 | 6/7/2001 | |
| | | | |
| **3656-1** | 150.3 | 5/30/2001 | Male |
| **3656-2** | 109.4 | 8/29/2001 | |
| **3656-3** | 109.7 | 9/12/2001 | |
| | | | |
| **3657-1** | 153.8 | 5/7/2001 | Male |
| **3657-2** | 122.3 | 6/6/2001 | |
| **3657-3** | 100.7 | 7/10/2001 | |
| | | | |
| **3658-1** | 75.8 | 4/17/2001 | Male |
| **3658-2** | 74.0 | 5/18/2001 | |
| **3658-3** | 70.8 | 6/21/2001 | |
| **3658-4** | 79.0 | 9/13/2001 | |
| | | | |
| **3659-1** | 82.7 | 7/6/2001 | Male |
| **3659-2** | 109.2 | 8/19/2001 | |
| **3659-3** | 99.7 | 9/24/2001 | |
| | | | |
| **3660-1** | 96.3 | 9/23/2001 | Male |
| **3660-2** | 146.3 | 10/16/2001 | |
| **3660-3** | 115.0 | 11/21/2001 | |
| | | | |
| **3661-1** | 172.0 | 8/7/2001 | Male |
| **3661-2** | 179.1 | 8/30/2001 | |
| **3661-3** | 149.5 | 10/10/2001 | |
| | | | |
| **3662-1** | 130.6 | 9/11/2001 | Male |
| **3662-2** | 116.7 | 10/1/2001 | |
| **3662-3** | 154.3 | 12/3/2001 | |
| | | | |
| **3663-1** | 75.5 | 8/22/2001 | Male |
| **3663-2** | 167.4 | 9/2012001 | |
| **3663-3** | 124.2 | 10/24/2001 | |
| | | | |
| **3664-1** | 67.6 | 5/9/2000 | Female |
| **3664-2** | 69.7 | 7/11/2000 | |
| **3664-3** | 40.2 | 8/9/2000 | |
| **3664-4** | 105.5 | 7/11/2001 | |
| | | | |
| **3665-1** | 86.4 | 3/13/2001 | Female |
| **3665-2** | 121.4 | 4/25/2001 | |
| **3665-3** | 86.1 | 6/20/2001 | |

### Example 6

### Single Point and Serial Ras Levels in Colon Cancer Patients

Ras is mutated in approximately 20% of all cancers, particularly in colon and pancreatic cancer. Based on the hypothesis of the invention, that a ras pathway activated by either mutation or overexpression may result in elevated levels of total ras p21 protein, 48 sera from colon cancer patients were assayed for total ras p21 levels (Table 7). At least four sera from colon cancer patients (samples # 2004, #2065, #2784 and #2785) showed extremely high total ras p21 levels.

**Table 7**

| Single Point Colorectal Cancer Patient Serum Samples (Ras Levels) | | | | |
|---|---|---|---|---|
| **Sample** | **Stage** | **Grade** | **Status** | **Ras p21 (pg/mL)** |
| 2000 | C2 | G3 | Prog | 135.09 |
| 2001 | C1 | 3 | Stab | 62.86 |
| 2002 | D | G4 | Recur | 57.86 |
| 2003 | C1 | 3 | Prog | 75.80 |
| 2004 | C2 | G2 | Recur | 1147.18 |
| 2005 | B2 | G1 | NED | 24.00 |
| 2006 | B2 | G1 | Prog | 38.39 |
| 2007 | C1 | 4 | P. Resp | 39.44 |
| 2008 | C2 | G3 | Prog | 121.38 |
| 2010 | C2 | 3 | Prog | 65.14 |
| 2011 | C2 | 3 | Prog | 105.74 |
| 2012 | C2 | 3 | Stab | 40.04 |
| 2013 | D | 4 | Prog | 118.44 |
| 2014 | C1 | 4 | P. Resp | 57.85 |
| 2015 | C2 | 3 | P. Resp | 98.02 |
| 2016 | D | G3 | Prog | 53.02 |
| 2017 | D | 4 | Prog | 113.46 |
| 2019 | C2 | 3 | Prog | 61.04 |
| 2020 | C1 | G1 | Prog | 49.69 |
| 2021 | D | G3 | Prog | 43.51 |
| 2022 | C1 | G1 | Prog | 84.97 |
| 2025 | D | G4 | Prog | 96.02 |
| 2026 | C2 | G2 | P. Resp | 54.98 |
| 2027 | D | G3 | Recur | 47.42 |
| 2028 | B2 | G2 | Stab | 73.05 |
| 2030 | C1 | G1 | P. Resp | 70.46 |
| 2034 | D | G3 | P. Resp | 76.25 |
| 2035 | C1 | G1 | Prog | 64.53 |
| 2036 | III | 2 | NED | 38.24 |
| 2038 | III | 2 | NED | 38.24 |
| 2052 | III | 2 | NED | 69.24 |
| 2054 | IV | 2 | Prog | 150.49 |
| 2055 | IV | 3 | Stab | 56.65 |
| 2056 | III | 2 | NED | 158.35 |
| 2057 | III | 2 | Prog | 137.91 |
| 2058 | IV | 3 | Prog | 90.18 |
| 2059 | IV | 3 | Stab | 48.03 |
| 2061 | IV | Unk | Stab | 198.64 |
| 2064 | IV | 4 | Prog | 107.74 |
| 2065 | IV | 4 | Prog | 319.91 |
| 2066 | IV | 4 | Prog | 138.55 |
| 2772 | III | Unk | Unk | 62.2 |
| 2773 | III | Unk | Unk | 157.8 |
| 2778 | III | Unk | Unk | 92.8 |
| 2781 | III | Unk | Unk | 209.4 |
| 2784 | III | Unk | Unk | 664.9 |
| 2785 | III | Unk | Unk | 1032.2 |
| 2786 | III | Unk | Unk | 137.5 |

However, serial changes in total ras p21 protein may be a more useful indicator of preneoplastic/neoplastic disease than a single point assay. Increasing levels of ras p21 may be indicative of colorectal cancer recurrence or progression and identify patients who should be treated with either conventional therapies or ras pathway targeted therapies. Increasing levels of ras oncoprotein may be used in conjunction with other oncology tests, such as carcinoembryonic antigen (CEA).

As shown in Table 8, serial serum samples from seven colon cancer patients were assayed for total ras p21 levels using the ELISA immunoassay format described in Materials and Methods. In one particular patient with stage IV colon cancer, the inventors made the following observations: the ras level initially remained relatively stable (Table 8, samples #3766-2 to #3766-4), but as the cancer progressed the ras levels increased to 69 pg/ml and then to 124 pg/ml. That patient showed progressive cancer while on conventional therapy. The immunoassay of the invention therefore may be used to identify a patient not responding to conventional therapy who may respond to therapies specifically targeted to inhibit the ras oncogene pathway or components of the pathway. The immunoassays of the invention may also be used to monitor response or lack of response to the ras pathway targeted therapies.

Another example is that of a stage II colon cancer patient, whose ras p21 level initially remained relatively stable (Table 8, samples #3767-1 to #3767-4). As the cancer progressed, however (samples #3767-5 and #3767-6), the ras level increased to 142 pg/ml and remained elevated at 95 pg/ml, while the patient's CEA level decreased to a normal 1.8 ng/ml. As shown in this patient's case, CEA decreased in the face of progressive disease, whereas ras levels increased, a pattern which is consistent with progressive disease. Elevated levels of ras may therefore be used to identify patients eligible for ras inhibitor therapies, or a patient not responding to conventional therapy that may be responsive to therapies specifically targeted to inhibit the ras oncogene pathway.

**Table 8**

| Serial serum samples from Colon Cancer patients (Ras p21 Levels) | | |
|---|---|---|
| Sample | Ras p21(pg/mL) | CEA (ng/mL) |
| **3766-2** | 59.4 | |
| **3766-3** | 43.7 | |
| **3766-4** | 52.8 | |
| **3766-5** | 69.3 | |
| **3766-6** | 124.8 | |
| | | |
| **3767-1** | 39.1 | 9.1 |
| **3767-2** | 46.5 | 6.5 |
| **3767-3** | 51.2 | 8.7 |
| **3767-4** | 38.5 | 6.4 |
| **3767-5** | 142.1 | 1.8 |
| **3767-6** | 95.6 | 1.8 |
| | | |
| **3768-1** | 50.1 | 3.8 |
| **3768-2** | 76.8 | 4.5 |
| **3768-3** | 53.4 | 2.9 |
| **3768-4** | 62.7 | 2.5 |
| **3768-5** | 63.3 | 2.7 |
| **3768-6** | 103.3 | 2.8 |
| | | |
| **3769-1** | 133.3 | |
| **3769-2** | 100.1 | < 0.7 |
| **3769-3** | 83.4 | < 0.7 |
| **3769-4** | 81.6 | 0.8 |
| **3769-5** | 75.4 | |
| **3769-6** | 87.1 | < 0.7 |
| | | |
| **3770-1** | 81.6 | 6.8 |
| **3770-2** | 97.5 | 7.4 |
| **3770-3** | 92.7 | 7.5 |
| **3770-4** | 61.5 | 9.2 |
| **3770-5** | 93.1 | 10.0 |
| **3770-6** | 117.0 | 10.0 |
| | | |
| **3771-1** | 43.5 | 26.7 |
| **3771-2** | 70.8 | 50.1 |
| **3771-3** | 50.9 | 81.0 |
| **3771-4** | 44.5 | 171.6 |
| **3771-5** | 41.3 | 368.7 |
| **3771-6** | 94.7 | 699.6 |
| | | |
| **3772-1** | 74.0 | 1.8 |
| **3772-2** | 50.8 | 5.8 |
| **3772-3** | 154.8 | 7.1 |
| **3772-4** | 50.2 | |
| **3772-5** | 83.4 | |
| **3772-6** | 158.0 | 20.0 |

### Example 7

### Ras p21 Serum Levels in Stage I/Stage II Breast Cancer

Ras p21 is overexpressed in 50-60% of breast cancers. Pretreatment elevated levels of the circulating ras oncoprotein in metastatic breast cancer (MBC) patients may indicate those patients eligible for either conventional or targeted anti-ras therapies.

In addition, increasing or decreasing levels of ras p21 alone, or in conjunction with levels of HER-2/neu, in breast cancer patients may be an early indicator of recurrent breast cancer. Pretreatment elevated circulating levels of ras or HER-2/neu oncoproteins in metastatic breast cancer (MBC) patients may indicate those patients eligible for anti-ras and/or anti-HER-2/neu therapies, either conventional or targeted therapies.

Table 9 shows total ras p21 levels found by immunoassay (as described under Materials and Methods) of single sera samples from Stage I or Stage II breast cancer patients. Individual breast cancer patients show high total ras p21 levels compared with normal levels in the 100-200 pg/ml range. Several patients show extremely high ras p21 levels in the 2600-2800 pg/ml range (see, for example, samples #1323, #1357 and #1432),

**Table 9**

| Single Point Stage I/II Breast Cancer Patient Serum Samples (Ras p21 Levels) | | | | |
|---|---|---|---|---|
| **Stage I** | | | **Stage II** | |
| **Breast Cancer Serums** | | | **Breast Cancer Serums** | |
| **Sample** | **Ras p21 (pg/mL)** | | **Sample** | **Ras p21 (pg/mL)** |
| 1318 | 312.9 | | 1406 | 99.6 |
| 1320 | 471.8 | | 1407 | 113.3 |
| 1321 | 2642.0 | | 1408 | 171.0 |
| 1322 | 53.0 | | 1409 | 82.6 |
| 1323 | 2646.0 | | 1410 | 76.2 |
| 1324 | 2648.0 | | 1411 | 140.2 |
| 1325 | 45.2 | | 1412 | 53.5 |
| 1326 | 60.0 | | 1413 | 83.6 |
| 1327 | 45.2 | | 1414 | 207.6 |
| 1328 | 324.6 | | 1415 | 76.7 |
| 1330 | 2660.0 | | 1416 | 291.3 |
| 1332 | 66.9 | | 1417 | 0.0 |
| 1333 | 74.5 | | 1418 | 50.5 |
| 1334 | 106.8 | | 1419 | 114.7 |
| 1335 | 89.6 | | 1420 | 2840.0 |
| 1336 | 2672.0 | | 1421 | 2842.0 |
| 1337 | 2674.0 | | 1422 | 59.2 |
| 1338 | 90.4 | | 1423 | 59.0 |
| 1339 | 56.3 | | 1424 | 106.3 |
| 1340 | 97.7 | | 1425 | 110.4 |
| 1341 | 66.9 | | 1426 | 51.0 |
| 1342 | 54.2 | | 1427 | 51.0 |
| 1343 | 50.3 | | 1428 | 2856.0 |
| 1344 | 65.1 | | 1429 | 138.5 |
| 1345 | 295.9 | | 1430 | 52.9 |
| 1346 | 77.2 | | 1431 | 2862.0 |
| 1348 | 61.5 | | 1432 | 2864.0 |
| 1349 | 261.6 | | 1433 | 44.9 |
| 1350 | 40.3 | | 1434 | 63.0 |
| 1351 | 2702.0 | | 1435 | 159.4 |
| 1352 | 62.8 | | 1436 | 111.7 |
| 1353 | 56.8 | | 1437 | 52.8 |
| 1354 | 2708.0 | | 1438 | 77.5 |
| 1355 | 50.5 | | 1439 | 74.5 |
| 1356 | 44.3 | | | |
| 1357 | 2714.0 | | | |
| 1358 | 2716.0 | | | |
| 1359 | 2718.0 | | | |
| 1362 | 101.6 | | | |
| 1365 | 232.4 | | | |
| 1366 | 181.0 | | | |
| 1367 | 85.1 | | | |

### Example 8

### Co-expression of Ras and TIMP-1 in Human Sera

Measuring ras p21 levels in combination with other tumor-associated proteins could aid in the selection of therapies for specific cancers. For example, by measuring levels of both ras and proteins upstream of ras, one may determine the most appropriate therapy for a neoplastic disease associated with an activated ras pathway.

One such upstream protein is TIMP-1 (tissue inhibitor of metalloproteinase-1). TIMP-1 is a multifunctional protein which is found in most tissues and body fluids; it inhibits the proteolytic activity of matrix metalloproteases and thereby prevents cancer invasion. However, TIMP-1 also possesses other functions such as inhibition of apoptosis, induction of malignant transformation and stimulation of cell-growth, and apparently activates ras. TIMP-1 is elevated in blood from colorectal cancer patients, and high TIMP-1 levels predict poor prognosis [38]. Monitoring levels of both ras and TIMP-1 would provide valuable information for therapy selection for a cancer patient, response or lack of response to therapy, as well as determining whether tumor progression is occurring.

Serial samples of ras p21 and TIMP-1 in colorectal cancer patients may be useful for predicting or monitoring response to targeted therapies (to ras and/or TIMP-1). There may also be a utility in measuring ras and TIMP-1 for predicting response to traditional anti-cancer therapies.

Pretreatment elevated ras p21 and TIMP-1 levels may be used for selecting patients for treatment with drugs meant to inhibit the ras and/or TIMP-1 proteins, or for predicting patient response to treatment to such drugs. Such treatment includes drugs that inhibit various components of the ras oncogene pathway, such as, raf kinase, and would apply to the raf kinase inhibitor drugs or to farnesyltransferase inhibitors (FTIs). Such treatment also includes conventional therapies used against ras driven tumors, or drugs meant to inhibit TIMP-1 function.

### Ras and TIMP-1 Levels in Serial Normal Samples

Serial serum samples from fifteen normal human controls (thirteen males, two females) were assayed by ELISA for total ras p21 and TIMP-1 levels as described above under Materials and Methods (shown in Table 10). In general, the samples were drawn at least three weeks apart. In the normal sera, total ras p21 levels varied between 50.6 pg/ml and 560 pg/ml; normal levels of TIMP-1 ranged between 167.8 ng/ml and 614.4 ng/ml.

**Table 10**

| Serial normal serum samples evaluated for ras p21 and TIMP-1 levels | | | | | |
|---|---|---|---|---|---|
| **Serial Normal Samples** | | | | | |
| | **Ras p21 (pg/mL)** | **TIMP-1 (ng/mL)** | | **Ras p21** | **TIMP-1** |
| **Sample** | | | **Sample** | **(pg/mL)** | **(ng/mL)** |
| 3651-1 | 159.0 | 342.9 | 3659-1 | 82.7 | 218.7 |
| 3651-2 | 221.1 | 368.5 | 3659-2 | 109.2 | 167.8 |
| 3651-3 | 141.3 | 214.4 | 3659-3 | 99.7 | 247.4 |
| | | | | | |
| 3652-1 | 50.6 | 330.7 | 3660-1 | 96.3 | 563.5 |
| 3652-2 | 56.0 | 395.0 | 3660-2 | 146.3 | 532.1 |
| 3652-3 | 58.8 | 279.8 | 3660-3 | 115.0 | 614.4 |
| | | | | | |
| 3653-1 | 246.7 | 201.4 | 3661-1 | 172.0 | 481.5 |
| 3653-2 | 560.0 | 192.9 | 3661-2 | 179.1 | 349.5 |
| 3653-3 | 244.4 | 269.0 | 3661-3 | 149.5 | 309.7 |
| | | | | | |
| 3654-1 | 113.9 | 354.7 | 3662-1 | 130.6 | 258.5 |
| 3654-2 | 127.9 | 324.1 | 3662-2 | 116.7 | 446.4 |
| 3654-3 | 170.2 | 399.2 | 3662-3 | 154.3 | 261.8 |
| 3654-4 | 126.7 | 471.9 | | | |
| | | | | | |
| 3655-1 | 101.0 | 465.0 | 3663-1 | 75.5 | 219.6 |
| 3655-2 | 99.6 | 445.9 | 3663-2 | 167.4 | 195.7 |
| 3655-3 | 132.3 | 430.8 | 3663-3 | 124.2 | 343.2 |
| | | | | | |
| 3656-1 | 150.3 | 368.6 | 3664-1 | 67.6 | 270.3 |
| 3656-2 | 109.4 | 398.3 | 3664-2 | 69.7 | 279.3 |
| 3656-3 | 109.7 | 336.2 | 3664-3 | 40.2 | 239.4 |
| | | | 3664-4 | 105.5 | 326.3 |
| 3657-1 | 153.8 | 351.5 | | | |
| 3657-2 | 122.3 | 375.3 | 3665-1 | 86.4 | 318.8 |
| 3657-3 | 100.7 | 238.4 | 3665-2 | 121.4 | 357.0 |
| | | | 3665-3 | 86.1 | 411.1 |
| 3658-1 | 75.8 | 282.3 | 3665-4 | | 369.5 |
| 3658-2 | 74.0 | 390.5 | | | |
| 3658-3 | 70.8 | 348.0 | | | |
| 3658-4 | 79.0 | 346.9 | | | |

### Ras p21, TIMP-1 and CEA Levels in Serial Colon Cancer Samples

As shown in Table 11, sera drawn serially from seven colon cancer patients were assayed by ELISA for total ras p21 and TIMP-1 levels as described above under Materials and Methods. Sera from six of those patients were also assayed for CEA.

In one particular stage IV colorectal cancer patient (Table 11, patient #3766), the ras level increased from 59 pg/ml to 125 pg/ml, whereas the TIMP-1 level increased from 263 to 703 ng/ml. The patient showed progressive disease while receiving conventional therapy. That data suggests that total ras p21 and TIMP-1 levels may be used in the management of such colorectal cancer patients.

**Table 11**

| Serial Colorectal Cancer Serum Samples Evaluated for ras p21 and TIMP-1 levels | | | | | | |
|---|---|---|---|---|---|---|
| *Sample* | Ras p21 (pg/mL) | CEA (ng/mL) | TIMP-1 (nq/mL) | Stage | Grade | Status |
| **3766-2** | 59.4 | | 263.2 | IV | 3 | Prog |
| **3766-3** | 43.7 | | 258.6 | IV | 3 | Prog |
| **3766-4** | 52.8 | | 323.3 | IV | 3 | Prog |
| **3766-5** | 69.3 | | 439.1 | IV | 3 | Prog |
| **3766-6** | 124.8 | | 702.7 | IV | 3 | Prog |
| | | | | | | |
| **3767-1** | 39.1 | 9.1 | 383.4 | II | 1 | Prog |
| **3767-2** | 46.5 | 6.5 | 415.3 | II | 1 | Prog |
| **3767-3** | 51.2 | 8.7 | 423.6 | II | 1 | Prog |
| **3767-4** | 38.5 | 6.4 | 438.2 | II | 1 | Prog |
| **3767-5** | 142.1 | 1.8 | 430.1 | II | 1 | Prog |
| **3767-6** | 95.6 | 1.8 | 534.5 | II | 1 | Prog |
| | | | | | | |
| **3768-1** | 50.1 | 3.8 | 390.2 | III | 2 | Stab |
| **3768-2** | 76.8 | 4.5 | 311.8 | III | 2 | Stab |
| **3768-3** | 53.4 | 2.9 | 409.5 | III | 2 | Stab |
| **3768-4** | 62.7 | 2.5 | 262.7 | III | 2 | Stab |
| **3768-5** | 63.3 | 2.7 | 302.1 | III | 2 | Stab |
| **3768-6** | 103.3 | 2.8 | 290.0 | III | 2 | Stab |
| | | | | | | |
| **3769-1** | 133.3 | | 212.7 | III | 1 | Stab |
| **3769-2** | 100.1 | < 0.7 | 197.6 | III | 1 | Stab |
| **3769-3** | 83.4 | < 0.7 | 219.6 | III | 1 | Stab |
| **3769-4** | 81.6 | 0.8 | 181.5 | III | 1 | Stab |
| **3769-5** | 75.4 | | 181.8 | III | 1 | Stab |
| **3769-6** | 87.1 | < 0.7 | 188.6 | III | 1 | Stab |
| | | | | | | |
| **3770-1** | 81.6 | 6.8 | 488.4 | III | 3 | Prog |
| **3770-2** | 97.5 | 7.4 | 527.7 | III | 3 | Prog |
| **3770-3** | 92.7 | 7.5 | 609.9 | III | 3 | Prog |
| **3770-4** | 61.5 | 9.2 | 724.9 | III | 3 | Prog |
| **3770-5** | 93.1 | 10.0 | 1210.8 | III | 3 | Prog |
| **3770-6** | 117.0 | 10.0 | 959.9 | III | 3 | Prog |
| | | | | | | |
| **3771-1** | 43.5 | 26.7 | 208.7 | III | 3 | Prog |
| **3771-2** | 70.8 | 50.1 | 282.2 | III | 3 | Prog |
| **3771-3** | 50.9 | 81.0 | 261.5 | III | 3 | Prog |
| **3771-4** | 44.5 | 171.6 | 322.6 | III | 3 | Prog |
| **3771-5** | 41.3 | 368.7 | 415.3 | III | 3 | Prog |
| **3771-6** | 94.7 | 699.6 | 468.8 | III | 3 | Prog |
| | | | | | | |
| **3772-1** | 74.0 | 1.8 | 265.8 | III | 2 | Prog |
| **3772-2** | 50.8 | 5.8 | 323.5 | III | 2 | Prog |
| **3772-3** | 154.8 | 7.1 | 471.1 | III | 2 | Prog |
| **3772-4** | 50.2 | | 460.5 | III | 2 | Prog |
| **3772-5** | 83.4 | | 491.5 | III | 2 | Prog |
| **3772-6** | 158.0 | 20.0 | 490.2 | III | 2 | Prog |

### Deposit of Hybridoma Cell Lines

ATCC Deposits. The material listed below was deposited with the American Type Culture Collection (ATCC) now at 10801 University Boulevard, Manassas, Va., 20110-2209 (USA). The deposits were made under the provisions of the Budapest Treaty on the International Recognition of Deposited Microorganisms for the Purposes of Patent Procedure and Regulations thereunder (Budapest Treaty). Maintenance of a viable culture is assured for thirty years from the date of deposit. The hybridomas and plasmids will be made available by the ATCC under the terms of the Budapest Treaty, and subject to an agreement between the Applicants and the ATCC which assures unrestricted availability of the deposited hybridomas and progeny thereof to the public upon issuance of a pertinent U.S. patent.

The assignee of the present application has agreed that if a culture of the material(s) on deposit should die or be lost or destroyed when cultivated under suitable conditions, to replace the material(s) promptly upon notification with another of the same.

| i. | Hybridoma | Deposit Date | ATCC # |
|---|---|---|---|
| ii. | Ras 10 | May 12, 1987 | HB 9426 |
| iii. | Ras 17 | May 29, 1989 | HB 10054 |

The description of the foregoing embodiments of the invention has been presented for purposes of illustration and description. They are not intended to be exhaustive or to limit the invention to the precise form disclosed, and obviously many modifications and variations are possible in light of the above teachings. The embodiments were chosen and described in order to explain the principles of the invention and its practical application to enable thereby others skilled in the art to utilize the invention in various embodiments and with various modifications as are suited to the particular use contemplated.

## Claims

1. A method to detect a preneoplastic/neoplastic disease associated with an activated ras pathway in a human subject comprising:
(a) immunologically detecting and quantifying the average level of total ras p21 protein comprising both normal and mutation activated ras in samples of a body fluid taken from individuals of a control population;
(b) immunologically detecting and quantifying serial changes in total ras p21 protein levels comprising both normal and mutation activated ras in equivalent samples of body fluid taken from the subject over time; and
(c) comparing the levels of total ras p21 protein in the subject's samples to the average level of total ras p21 protein in the control samples;
wherein a level of total ras p21 protein in the subject's samples that is above the average level of total ras p21 protein in the control samples is indicative of an activated ras pathway and the presence of preneoplastic/neoplastic disease in the subject, and wherein said immunological detection and quantification of steps (a) and (b) is by an immunoassay in the form of a sandwich immunoassay using a combination of the monoclonal antibody Ras 17 which is secreted from hybridoma HB 10054 deposited at the American Type Culture Collection (ATCC), and the monoclonal antibody Ras 10 which is secreted from hybridoma HB 9426 deposited at the ATCC.

2. The method of claim 1 which is further prognostic for said preneoplastic/neoplastic disease, wherein said levels of total ras p21 protein in the subject's samples relative to the average level of total ras p21 in the control samples, are indicative of a better or poorer prognosis for said subject.

3. The method of claim 1 for use in therapy selection for a human patient with a preneoplastic/neoplastic disease further comprising:
(d) deciding whether to use conventional cancer therapy and/or ras-directed cancer therapy to treat the patient based upon the differences between the levels of total ras p21 protein in the patient's samples and the average level of total ras p21 protein in the control samples, and in view of the serial changes among the levels of total ras p21 protein in the patient's samples.

4. The method of claim 3, wherein if a level of total ras p21 protein in a patient's sample is found to be above the average level of total ras p21 protein in the control samples, and if the serial changes in the total ras p21 protein levels in the patient's samples reflect levels that are trending above the average level of total ras p21 protein in the control samples, concluding that the patient has a ras oncogene driven preneoplastic/neoplastic disease, and deciding to use a ras-directed therapy to treat the patient.

5. The method of claim 3 which is further prognostic for said preneoplastic/neoplastic disease, wherein said levels of total ras p21 protein in the subject's samples relative to the average level of total ras p21 in the control samples are indicative of a better or poorer prognosis for said subject, and wherein said decision in step (d) is based on said prognosis.

6. The method of claim 3, wherein said ras-directed therapy is selected from the group consisting of farnesyltransferase inhibitors (FTI), tyrosine kinase inhibitors, bis-aryl ureas, and antisense inhibitors of ras.

7. The method of claim 6, wherein said ras-directed therapy is the bis aryl-urea sorafenib (BAY 43-9006).

8. The method of claims 1, wherein said individuals of the control population of step (a) are of the same gender as the subject.

9. The method of claim 1, wherein the detector monoclonal antibody Ras 10 is biotinylated.

10. The method of claim 3, wherein the body fluid samples are from a cancer patient who has not responded to treatment.

11. A method of monitoring the status of a preneoplastic/neoplastic disease in a patient, and/or monitoring how a patient with a preneoplastic/neoplastic disease is responding to a therapy, comprising immunologically detecting and quantifying serial changes in total ras p21 protein levels comprising both normal and mutation activated ras in samples of a body fluid taken from said patient over time;
wherein increasing levels of total ras p21 protein over time indicate disease progression or a negative response to said therapy, and wherein decreasing levels of total ras p21 protein indicate disease remission or a positive response to said therapy,
wherein said immunological detection and quantification of steps is by an immunoassay in the form of a sandwich immunoassay using a combination of the monoclonal antibody Ras 17 which is secreted from hybridoma HB 10054 deposited at the American Type Culture Collection (ATCC), and the monoclonal antibody Ras 10 which is secreted from hybridoma HB 9426 deposited at the ATCC.

12. The method of claim 11, wherein said therapy is a ras-directed therapy.

13. The method of claim 11 which is further prognostic for said preneoplastic/neoplastic disease, wherein said levels of total ras p21 protein in the subject's samples are indicative of a better or poorer prognosis for said subject.

14. The method of claims 2, 5 and 13, wherein said prognosis is a clinical outcome selected from the group consisting of response rate (RR), complete response clinical benefit (CR), partial response clinical benefit (PR), stable disease clinical benefit (SO), time to progression (TTP), and time to death (TTO).

15. The method of claims 2, 3 and 11, wherein said samples taken from said subject or from said patient are pretreatment samples.

16. The method of claims 1, 3 and 11, further comprising the use of an immunoassay to detect or detect and quantify levels of one or more other proteins in the patient's samples.

17. The method of claim 16, wherein said other protein is or said other proteins are selected from the group consisting of inhibitors, oncoproteins, growth factor receptors, angiogenic factors, metastasis proteins, tumor markers, and tumor suppressors.

18. The method of claim 17, wherein said inhibitor is tissue inhibitor of metalloproteinase-1 (TIMP-1), said oncoprotein is HER-2/neu, said growth factor receptors are selected from the group consisting of epidermal growth factor receptor (EGFR) and platelet derived growth factor receptor (POGFR), said angiogenic factor is vascular endothelial growth factor (VEGF), said metastasis protein is urokinase-type plasminogen activator (uPA), said tumor marker is carcinoembryonic antigen (CEA), and said tumor suppressor is p53.

19. The method of claim 11, wherein the body fluid samples are from a cancer patient who has not responded to treatment.

20. The method of claim 19, wherein increasing levels of total ras p21 and/or HER-2/neu are indicative of a greater probability of early recurrence or metastasis.

21. The method of claims 1, 3 and 11, wherein said body fluid is selected from the group consisting of blood, serum, plasma, urine, saliva, semen, breast exudate, cerebrospinal fluid, tears, sputum, mucous, Iymph, cytosols, ascites, pleural effusions, amniotic fluid, bladder washes and bronchioalveolar lavages.

22. The method of claims 1, 3 and 11, wherein said body fluid is serum or plasma.

23. The method of claims 1, 3 and 11, wherein said preneoplastic/neoplastic disease associated with an activated ras pathway is selected from the group consisting of colorectal-cancer, colon cancer, lung cancer, non-small-cell lung cancer, small-cell lung cancer, acute myelogenous leukemia, thyroid cancer, pancreatic cancer, bladder cancer, kidney cancer, melanoma, breast cancer, prostate cancer, ovarian cancer, cervical cancer, head-and-neck cancer, hepatocellular carcinoma and hematologic malignancy.

## Patentansprüche

1. Verfahren zum Erkennen präneoplastischer/neoplastischer Erkrankungen, die mit einem aktivierten Ras-Signalweg in einem menschlichen Subjekt einhergehen, wobei das Verfahren folgendes umfasst:
(a) immunologisches Erkennen und Quantifizieren der durchschnittlichen Menge an Gesamt-Ras-p21-Protein, umfassend sowohl normales als auch durch Mutation aktiviertes Ras in Proben einer Körperflüssigkeit, die Individuen einer Kontrollpopulation entnommen wurden;
(b) immunologisches Erkennen und Quantifizieren serieller Veränderungen der Mengen an Gesamt-Ras-p21-Protein, umfassend sowohl normales als auch durch Mutation aktiviertes Ras in äquivalenten Proben einer Körperflüssigkeit, die dem Subjekt über einen Zeitraum hinweg entnommen wurden; und
(c) Vergleichen der Mengen an Gesamt-Ras-p21-Protein in den Proben von dem Subjekt mit der durchschnittlichen Menge an Gesamt-Ras-p21-Protein in den Kontrollproben;
wobei eine Menge an Gesamt-Ras-p21-Protein in den Proben von dem Subjekt, die oberhalb der durchschnittlichen Menge an Gesamt-Ras-p21-Protein in den Kontrollproben liegt, einen Hinweis auf einen aktivierten Ras-Signalweg und das Vorliegen einer präneoplastischen/neoplastischen Erkrankung in dem Subjekt liefert und wobei die besagte immunologische Erkennung und Quantifizierung der Schritte (a) und (b) durch ein Immunoassay in Form eines Sandwich-Immunoassays erfolgt, wozu eine Kombination des monoklonalen Antikörpers Ras 17, der von dem HB-10054-Hybridom, das bei der American Type Culture Collection (ATCC) hinterlegt ist, sekretiert wurde, und dem monoklonalen Antikörper Ras 10, der von dem bei der ATCC hinterlegten HB-9426-Hybridom sekretiert wurde, verwendet wird.

2. Verfahren gemäß Anspruch 1, das ferner eine Prognose zu der besagten präneoplastischen/neoplastischen Erkrankung liefert, wobei die besagten Mengen an Gesamt-Ras-p21-Protein in den Proben von dem Subjekt bezogen auf die durchschnittliche Menge an Gesamt-Ras-p21-Protein in den Kontrollproben Hinweise für eine bessere oder schlechtere Prognose für das besagte Subjekt geben.

3. Verfahren gemäß Anspruch 1 zum Einsatz bei der Auswahl einer Therapie für einen menschlichen Patienten mit einer präneoplastischen/neoplastischen Erkrankung, das ferner folgendes umfasst:
(d) Entscheiden, ob eine konventionelle Krebstherapie und/oder eine gegen das Ras gerichtete Krebstherapie zur Behandlung des Patienten eingesetzt werden soll, basierend auf den Unterschieden zwischen den Mengen an Gesamt-Ras-p21-Protein in den Proben von dem Patienten und der durchschnittlichen Menge an Gesamt-Ras-p21-Protein in den Kontrollproben sowie unter Berücksichtigung der seriellen Veränderungen der Mengen an Gesamt-Ras-p21-Protein in den Proben von dem Patienten.

4. Verfahren gemäß Anspruch 3, wobei, wenn festgestellt wird, dass eine Menge an Gesamt-Ras-p21-Protein in einer Probe von dem Patienten oberhalb der durchschnittlichen Menge an Gesamt-Ras-p21-Protein in den Kontrollproben liegt, und wenn die seriellen Veränderungen der Mengen an Gesamt-Ras-p21-Protein in den Proben von dem Patienten Mengen widerspiegeln, die tendenziell oberhalb der durchschnittlichen Menge an Gesamt-Ras-p21-Protein in den Kontrollproben liegen, schlussfolgern, dass bei dem Patienten eine durch ein Ras-Onkogen verursachte präneoplastische/neoplastische Erkrankung vorliegt, und entscheiden, dass eine gegen das Ras gerichtete Therapie für die Behandlung des Patienten angewandt wird.

5. Verfahren gemäß Anspruch 3, welches ferner eine Prognose zu der besagten präneoplastischen/neoplastischen Erkrankung liefert, wobei die besagten Mengen an Gesamt-Ras-p21-Protein in den Proben von dem Subjekt bezogen auf die durchschnittliche Menge an Gesamt-Ras-p21-Protein in den Kontrollproben Hinweise für eine bessere oder schlechtere Prognose für das besagte Subjekt geben und wobei sich die besagte Entscheidung in Schritt (d) auf die besagte Prognose stützt.

6. Verfahren gemäß Anspruch 3, wobei die besagte gegen das Ras gerichtete Therapie aus einer Gruppe ausgewählt wird, die aus Farnesyl-Transferase-Inhibitoren (FTI), Tyrosin-Kinase-Inhibitoren, Biaryl-Harnstoff und Antisense-Inhibitoren von Ras besteht.

7. Verfahren gemäß Anspruch 6, wobei die besagte gegen das Ras gerichtete Therapie der Biaryl-Harnstoff Sorafenib (BAY 43-9006) ist.

8. Verfahren gemäß Anspruch 1, wobei die besagten Individuen der Kontrollpopulation von Schritt (a) dasselbe Geschlecht haben wie das Subjekt.

9. Verfahren gemäß Anspruch 1, wobei der als Detektor verwendete monoklonale Antikörper Ras 10 biotinyliert ist.

10. Verfahren gemäß Anspruch 3, wobei die Proben von Körperflüssigkeit von einem Krebspatienten stammen, der auf die Behandlung nicht angesprochen hat.

11. Verfahren zum Überwachen des Status einer präneoplastischen/neoplastischen Erkrankung bei einem Patienten und/oder zum Überwachen, wie ein Patient mit einer präneoplastischen/neoplastischen Erkrankung auf eine Therapie anspricht, wobei das Verfahren das immunologische Erkennen und Quantifizieren serieller Veränderungen der Mengen an Gesamt-Ras-p21-Protein, umfassend sowohl normales als auch durch Mutation aktiviertes Ras in Proben einer Körperflüssigkeit, die dem besagten Patienten über einen Zeitraum hinweg entnommen wurden, umfasst;
wobei über den betreffenden Zeitraum hinweg steigende Mengen an Gesamt-Ras-p21-Protein ein Fortschreiten der Erkrankung oder ein negatives Ansprechen auf die besagte Therapie anzeigen und wobei abnehmende Mengen an Gesamt-Ras-p21-Protein ein Zurückgehen der Erkrankung oder ein positives Ansprechen auf die besagte Therapie anzeigen,
wobei die besagte immunologische Erkennung und Quantifizierung der Schritte durch ein Immunoassay in Form eines Sandwich-Immunoassays erfolgt, wozu eine Kombination des monoklonalen Antikörpers Ras 17, der von dem HB-10054-Hybridom, das bei der American Type Culture Collection (ATCC) hinterlegt ist, sekretiert wurde, und dem monoklonalen Antikörper Ras 10, der von dem bei der ATCC hinterlegten HB-9426-Hybridom sekretiert wurde, verwendet wird.

12. Verfahren gemäß Anspruch 11, wobei die besagte Therapie eine gegen das Ras gerichtet Therapie ist.

13. Verfahren gemäß Anspruch 11, das ferner eine Prognose zu der besagten präneoplastischen/neoplastischen Erkrankung liefert, wobei die besagten Mengen an Gesamt-Ras-p21-Protein in den Proben von dem Subjekt Hinweise für eine bessere oder schlechtere Prognose für das besagte Subjekt geben.

14. Verfahren gemäß den Ansprüchen 2, 5 und 13, wobei die besagte Prognose ein klinisches Ergebnis ist, das aus der Gruppe ausgewählt wurde, die aus der Ansprechrate (RR, Response Rate), dem klinischen Nutzen eines vollständigen Ansprechens des Tumors (CR, Complete Response), dem klinischen Nutzen eines teilweisen Ansprechens des Tumors (PR, Partial Response), dem klinischen Nutzen einer Stabilisierung der Erkrankung (SD, Stable Disease), Zeit bis zur Progression (TTP, Time to Progression) und Zeit bis zum Tod (TTD, Time to Death) besteht.

15. Verfahren gemäß den Ansprüchen 2, 3 und 11, wobei die besagten Proben, die dem besagten Subjekt oder dem besagten Patienten entnommen wurden, Proben vor Behandlung sind.

16. Verfahren gemäß den Ansprüchen 1, 3 und 11, das ferner die Verwendung eines Immunoassays umfasst, um die Mengen eines anderen Proteins oder mehrerer anderer Proteine in den Proben von dem Patienten zu erkennen oder zu erkennen und zu quantifizieren.

17. Verfahren gemäß Anspruch 16, wobei das besagte andere Protein bzw. die besagten anderen Proteine aus der Gruppe ausgewählt ist/sind, die aus Inhibitoren, Onkoproteinen, Wachstumsfaktor-Rezeptoren, angiogenen Faktoren, Metastasierungsproteinen, Tumormarkern und Tumorsuppressoren besteht.

18. Verfahren gemäß Anspruch 17, wobei der besagte Inhibitor Gewebe-Inhibitor der Metallproteinase-1 (TIMP-1) ist, das besagte Onkoprotein Her-2/neu ist, die besagten Wachstumsfaktor-Rezeptoren aus der Gruppe, die aus dem epidermalen Wachstumsfaktor-Rezeptor (EGFR) und dem thrombozytären Wachstumsfaktor-Rezeptor (PDGFR) besteht, ausgewählt sind, der besagte angiogene Faktor der vaskuläre endotheliale Wachstumsfaktor (VEGF) ist, das besagte Metastasierungs-Protein ein urokinaseartiger Plasminogenaktivator (uPA) ist, der besagte Tumormarker ein carzinoembryonales Antigen (CEA) ist und der besagte Tumorsuppressor p53 ist.

19. Verfahren gemäß Anspruch 11, wobei die Proben der Körperflüssigkeit von einem Krebspatienten stammen, der auf die Behandlung nicht angesprochen hat.

20. Verfahren gemäß Anspruch 19, wobei ansteigende Mengen an Gesamt-Ras-p21 und/oder HER-2/neu auf eine größere Wahrscheinlichkeit des frühzeitigen Wiederauftretens bzw. der Metastasierung hinweisen.

21. Verfahren gemäß den Ansprüchen 1, 3 und 11, wobei die besagte Körperflüssigkeit aus der Gruppe ausgewählt ist, die aus Blut, Serum, Plasma, Urin, Speichel, Sperma, Brustexudat, Gehirn-Rückenmark-Flüssigkeit, Tränenflüssigkeit, Sputum, Schleim, Lymphflüssigkeit, Zellplasma, Asciten, Pleuraergüssen, Fruchtwasser, Blasenspülungen und bronchoveolären Lavagen besteht.

22. Verfahren gemäß den Ansprüchen 1, 3 und 11, wobei die besagte Körperflüssigkeit Serum oder Plasma ist.

23. Verfahren gemäß den Ansprüchen 1, 3 und 11, wobei die besagte präneoplastische/neoplastische Erkrankung, die mit einem aktivierten Ras-Signalweg einhergeht, aus der Gruppe ausgewählt ist, die aus kolorektalem Krebs, Darmkrebs, Lungenkrebs, nicht-kleinzelligem Lungenkrebs, kleinzelligem Lungenkrebs, akuter myeloischer Leukämie, Schilddrüsenkrebs, Bauchspeicheldrüsenkrebs, Blasenkrebs, Nierenkrebs, schwarzem Hautkrebs, Brustkrebs, Prostatakrebs, Eierstockkrebs, Gebärmutterhalskrebs, Kopf-Hals-Krebs, Leberzellkrebs und hämatologischer Malignität besteht.

## Revendications

1. Procédé de détection d'une maladie prénéoplastique/ néoplastique associée à une voie de ras activée chez un sujet humain, dans lequel :
(a) On détecte et on quantifie immunologiquement le taux moyen de protéine de ras p21 totale comprenant à la fois de la ras normale et de la ras activée par mutation dans des échantillons d'un fluide corporel prélevé chez des individus d'une population témoin ;
(b) On détecte et on quantifie immunologiquement des variations successives des taux totales de protéines de ras p21 totale, comprenant à la fois du ras normal et du ras activé par mutation, dans des échantillons équivalents de fluide corporel prélevés chez le sujet au cours du temps ; et
(c) On compare les taux de protéine totale de ras p21 dans les échantillons du sujet au taux moyen de protéine de ras p21 totale dans les échantillons témoins ;
Un taux de protéine de ras p21 totale dans les échantillons du sujet, qui est au-dessus du taux moyen de protéine de ras p21 totale dans les échantillons témoins, indiquant un ras activé et la présence d'une maladie prénéoplastique/ néoplastique chez le sujet et la détection immunologique et la quantification des stades ( a ) et ( b ) s'effectuent par un immunodosage sous la forme d'un immunodosage sandwich utilisant une combinaison de l'anticorps monoclonal Ras 17, qui est sécrété par l'hybridome HB 10054 déposé à l'American Type Culture Collection ( ATCC ), et de l'anticorps monoclonal Ras 10, qui est sécrété par l'hybridome HB 9426 déposé à l'ATCC.

2. Procédé suivant la revendication 1 qui est en outre un pronostic de la maladie prénéoplastique/néoplastique, les taux de protéine de ras p21 totale dans les échantillons du sujet par rapport au taux moyen de ras p21 totale dans les échantillons témoins indiquant un pronostic meilleur ou moins bon pour le sujet.

3. Procédé suivant la revendication 1, à utiliser en sélection de thérapie pour un patient humain ayant une maladie prénéoplastique/néoplastique dans lequel en outre :
(d) On décide si on utilise une thérapie classique du cancer et/ou une thérapie du cancer visant ras pour traiter le patient sur la base des différences entre les taux de protéine de ras p21 totale dans les échantillons du patient et le taux moyen de protéine de ras p21 totale dans les échantillons témoins et au vu des variations successives entre les taux de protéines de ras p21 totales dans les échantillons du patient.

4. Procédé suivant la revendication 3, dans lequel, si on trouve qu'un taux de protéine de ras p21 totale dans un échantillon du patient est au dessus du taux moyen de protéine de ras p21 totale dans les échantillons témoins et si les variations successives des taux de protéine de ras p21 totale dans les échantillons du patient réflectent des taux qui tendent à être supérieurs au taux moyens de protéine de ras p21 totale dans les échantillons témoins, on conclue que le patient a une maladie prénéoplastique/néoplastique par oncogène ras et on décide d'utiliser une thérapie visant ras pour traiter le patient.

5. Procédé suivant la revendication 3, qui est en outre un pronostic de la maladie prénéoplastique/néoplastique, dans lequel les taux de protéine de ras p21 totale dans les échantillons du sujet par rapport au taux moyen de ras p21 totale dans les échantillons témoins indiquent un pronostic meilleur ou moins bon pour le sujet et la décision du stade ( d ) repose sur ce pronostic.

6. Procédé suivant la revendication 3, dans lequel on choisit la thérapie visant ras dans le groupe consistant en des inhibiteurs de farnesyltransférase ( FTI ) des inhibiteurs de tyrosine kinase, des bis-aryl urées et des inhibiteurs anti-sens de ras.

7. Procédé suivant la revendication 6, dans lequel la thérapie visant ras est le bis aryl urée sorafenib ( BAY 43-9006 ).

8. Procédé suivant la revendication 1, dans lequel les individus de la population témoin du stade ( a ) sont du même genre que le sujet.

9. Procédé suivant la revendication 1, dans lequel l'anticorps monoclonal de détecteur Ras 10 est biotinylé.

10. Procédé suivant la revendication 3, dans lequel les échantillons de fluide corporel proviennent d'un patient cancéreux qui n'a pas réagi à un traitement.

11. Procédé de control de l'état d'une maladie prénéoplastique/néoplastique chez un patient et/ou de controle de la façon dont un patient ayant une maladie prénéoplastique/néoplastique réagit à une thérapie, dans lequel on détecte et quantifie immunologiquement des variations successives des taux de protéine de ras p21 totale, comprenant à la fois de la ras normale et de la ras activée par mutation, dans des échantillons d'un fluide corporel prélevé du patient au cours du temps ;
des taux croissants de protéine de ras p21 totale en fonction du temps indiquant une progression de la maladie ou une réaction négative à la thérapie et des taux décroissants de protéine de ras p21 totale indiquant une rémission de la maladie ou une réaction positive à la thérapie,
la détection et la quantification immunologique des stades s'effectuant par un immunodosage sous la forme d'un immunodosage sandwich, utilisant une combinaison de l'anticorps monoclonal Ras 17, qui est sécrété par l'hybridome HB 10054 déposé à l'Américan Type Culture Collection ( ATCC ), et de l'anticorps monoclonal Ras 10, qui est sécrété par l'hybridome HB 9426 déposé à l'ATCC.

12. Procédé suivant la revendication 11, dans lequel la thérapie est une thérapie visant ras.

13. Procédé suivant la revendication 11, qui est en outre un pronostic de la maladie prénéoplastique/néoplastique les taux de protéine de ras p21 totale dans les échantillons du sujet par rapport au taux moyen de ras p21 totale dans les échantillons témoins indiquant un pronostic meilleur ou moins bon pour le sujet.

14. Procédé suivant les revendications 2, 5 et 13, dans lequel le pronostic est un résultat clinique choisi dans le groupe consistant en une vitesse de réaction ( RR ), un bénéfice clinique de réaction complète ( CR ), un bénéfice clinique de réaction partielle ( PR ), un bénéfice clinique de maladie stable ( SO ), une durée d'évolution ( TTP ) et une durée jusqu'à la mort ( TTO ).

15. Procédé suivant les revendications 2, 3 et 11 dans lequel les échantillons prélevés chez le sujet ou chez le patient sont des échantillons de pré-traitement.

16. Procédé suivant les revendications 1, 3 et 11, comprenant en outre l'utilisation d'un immunodosage pour détecter ou pour détecter et quantifier des taux d'une autre protéine ou de plusieurs autres protéines dans les échantillons du patient.

17. Procédé suivant la revendication 16, dans lequel l'autre protéine ou les autres protéines est ou sont choisies dans le groupe consistant en des inhibiteurs, en des onco-protéines, en des récepteurs de facteur de croissance, en des facteurs angiogéniques, en des protéines de métastases, en des marqueurs de tumeur et en des suppresseurs de tumeur.

18. Procédé suivant la revendication 17, dans lequel l'inhibiteur est un inhibiteur tissulaire de métalloprotéinase-1 ( TIMP-1 ), l'onco-protéine est HER-2/nouveau, les récepteurs de facteur de croissance sont choisis dans le groupe consistant en un récepteur de facteur de croissance épidermique ( EGFR ) et en un récepteur de facteur de croissance dérivé des plaquettes ( POGFR ), le facteur angiogène est un facteur de croissance endothélial vasculaire ( VEGF ), la protéine de métastase est un activateur de plasminogène de type urokinase ( uPA ), le marqueur de tumeur est un antigène carcinoembryonique ( CEA ), et le suppresseur de tumeur est P53.

19. Procédé suivant la revendication 11, dans lequel les échantillons de fluide corporel proviennent d'un patient cancéreux qui n'a pas régi au traitement.

20. Procédé suivant la revendication 19, dans lequel des taux croissants de ras p21 totale et/ou de HER-2/nouveau indique une probabilité plus grande de rechute précoce ou de métastase.

21. Procédé suivant la revendication 1, 3 et 11, dans lequel on choisit le fluide corporel dans le groupe consistant en le sang, le sérum, le plasma, l'urine, la salive, le sperme, l'exsudat du sein, le fluide cérébrospinal, les larmes, l'expectoration, la muqueuse, la lymphe, les cytosols, les ascites, les épanchements pleuraux, le fluide amniotique, les lavages vésicaux et les lavages bronchioalvéolaires.

22. Procédé suivant la revendication 1, 3 et 11, dans lequel le fluide corporel est le sérum ou le plasma.

23. Procédé suivant la revendication 1, 3 et 11, dans lequel la maladie prénéoplastique/néoplastique associée à une voie ras activée est choisie dans le groupe consistant en le cancer du colon et du rectum, le cancer du colon, le cancer du poumon, le cancer du poumon à cellules non petites, le cancer du poumon à cellules petites, la leucémie myéloblastique aigue, le cancer de la tyroïde, le cancer du pancréas, le cancer de la vessie, le cancer du rein, le mélanome, le cancer du sein, le cancer de la prostate, le cancer des ovaires, le cancer du col, le cancer de la tête et du cou, l'hépatome et l'affection maligne hématologique.
